(19) Europäisches Patentamt
European Patent Office
Office européen des brevets

(11) Publication number : **0 381 989 B1**

(12) **EUROPEAN PATENT SPECIFICATION**

(45) Date of publication of patent specification : **05.02.92 Bulletin 92/06**

(51) Int. Cl.$^5$ : **C07H 15/252,** A61K 31/70

(21) Application number : **90101309.4**

(22) Date of filing : **23.01.90**

(54) **New 4'-epi-4'-amino anthracyclines.**

(30) Priority : **07.02.89 GB 8902709**

(43) Date of publication of application : **16.08.90 Bulletin 90/33**

(45) Publication of the grant of the patent : **05.02.92 Bulletin 92/06**

(84) Designated Contracting States : **AT BE CH DE DK ES FR GB GR IT LI NL SE**

(56) References cited :
**EP-A- 0 051 280**
**EP-A- 0 328 400**
**GB-A- 2 091 243**
**BIOMEDICAL AND ENVIRONMENTAL MASS SPECTROMETRY, vol. 13, no. 7, 1986, pages 319-326, John Wiley & Sons Ltd; C. MONNERET et al.: "Desorption chemical ionization mass spectrometry of anthracyclines and of trisaccharides related to aclacinomycin A and marcellomycin"**

(73) Proprietor : **FARMITALIA CARLO ERBA S.r.L.**
**Via Carlo Imbonati 24**
**I-20159 Milano (IT)**

(72) Inventor : **Angelucci, Francesco**
**Via Washington, 106**
**I-20100 Milan (IT)**
Inventor : **Bargiotti, Alberto**
**Via Donati, 8**
**I-20100 Milan (IT)**
Inventor : **Faiardi, Daniela**
**Via Cassinino, 42**
**I-27100 Pavia (IT)**
Inventor : **Stefanelli, Stefania**
**Via San Giuseppe Cottolengo, 42**
**I-20143 Milan (IT)**
Inventor : **Suarato, Antonino**
**Via Degli Imbriani, 39**
**I-20100 Milan (IT)**

(74) Representative : **Giambrocono, Alfonso, Dr. Ing. et al**
**Ing. A. Giambrocono & C. S.r.l. Via Rosolino Pilo 19/B**
**I-20129 Milano (IT)**

**EP 0 381 989 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a new class of anthracycline glycosides having antitumour activity, methods for their preparation, pharmaceutical compositions containing them and the use thereof in treating certain mammalian tumours. The invention also relates to the preparation of certain novel intermediates.

The invention provides anthracycline glycosides having the general formulae 1 and 2 :

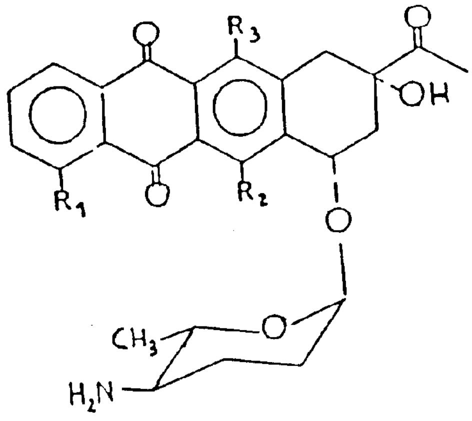

1

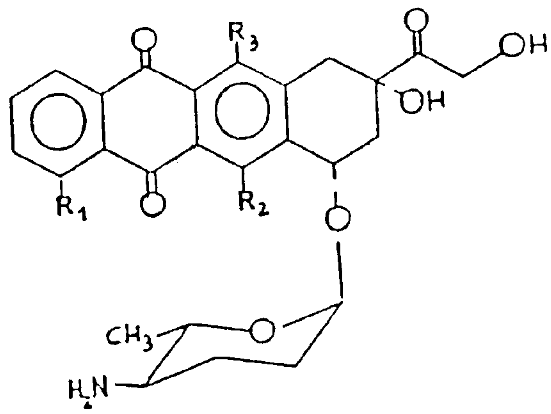

2

wherein $R_1$ is selected from the group consisting of hydrogen, fluorine, hydroxy and amino ; $R_2$ and $R_3$ both represent hydroxy or one of $R_2$ and $R_3$ is hydrogen, nitro or amino and the other of $R_2$ and $R_3$ is hydroxy ; with the proviso that when $R_2$ and $R_3$ are both hydroxy, then $R_1$ is fluorine, hydroxy or amino ; and pharmaceutically acceptable salts thereof. Preferred salts are the hydrochloride salts.

3-Deamino-4′-deoxy-4′-amino anthracyclines are described in the Applicant's previosuly filed EP-A-0328400. Said class of anthracycline glycosides was obtained by condensation of a suitable sugar derivative, wherein the amino group in 4′-position is either in axial or equatorial configuration, with daunomycinone or 4-demethoxydaunomycinone.

In the novel synthetized anthracycline glycosides, hereinafter described, the aglycone moiety has a different framework owing to the different substituents either in 4 or in 6 and 11 position.

The anthracycline glycosides of the general formulae 1 and 2 include :

4-demethyl-3′-deamino-4′-deoxy-4′-epi-amino-daunorubicin
(1a : $R_1$=$R_2$=$R_3$=OH)
4-demethyl-3′-deamino-4′-deoxy-4′-epi-amino-doxorubicin
(2a : $R_1$=$R_2$=$R_3$=OH)
4-demethoxy-4-amino-3′-deamino-4′-deoxy-4′-epi-amino-daunorubicin
(1b : $R_1$=$NH_2$, $R_2$=$R_3$=OH)
4-demethoxy-4-amino-3′-deamino-4′-deoxy-4′-epi-amino-doxorubicin
(2b : $R_1$=$NH_2$, $R_2$=$R_3$=OH)
4-demethoxy-4-fluoro-3′-deamino-4′-deoxy-4′-epi-amino-daunorubicin
(1c : $R_1$=F, $R_2$=$R_3$=OH)
4-demethoxy-4-fluoro-3′-deamino-4′-deoxy-4′-epi-amino-doxorubicin
(2c : $R_1$=F, $R_2$=$R_3$=OH)
4-demethyl-6-deoxy-3′-deamino-4′-deoxy-4′-epi-amino-daunorubicin
(1d : $R_1$=$R_3$=OH, $R_2$=H)
4-demethyl-6-deoxy-3′-deamino-4′-deoxy-4′-epi-amino-doxorubicin
(2d : $R_1$=$R_3$=OH, $R_2$=H)
4-demethoxy-11-deoxy-11-nitro-3′-deamino-4′-deoxy-4′-epi-amino-daunorubicin
(1e : $R_1$=H, $R_2$=OH, $R_3$=$NO_2$)
4-demethoxy-11-deoxy-11-nitro-3′-deamino-4′-deoxy-4′-epi-amino-doxorubicin
(2e : $R_1$=H, $R_2$=OH, $R_3$=$NO_2$)
4-demethoxy-6-deoxy-6-nitro-3′-deamino-4′-deoxy-4′-epi-amino-daunorubicin
(1f : $R_1$=H, $R_2$=$NO_2$, $R_3$=OH)

4-demethoxy-6-deoxy-6-nitro-3′-deamino-4′-deoxy-4′-epi-amino-doxorubicin
(2f : $R_1$=H, $R_2$=$NO_2$, $R_3$=OH)
4-demethoxy-11-deoxy-11-amino-3′-deamino-4′-deoxy-4′-epi-amino-daunorubicin
(1g : $R_1$=H, $R_3$=$NH_2$, $R_2$=OH)
4-demethoxy-11-deoxy-11-amino-3′-deamino-4′-deoxy-4′-epi-amino-doxorubicin
(2g : $R_1$=H, $R_3$=$NH_2$, $R_2$=OH)
4-demethoxy-6-deoxy-6-amino-3′-deamino-4′-deoxy-4′-epi-amino-daunorubicin
(1h : $R_1$=H, $R_2$=$NH_2$, $R_3$=OH)
4-demethoxy-6-deoxy-6-amino-3′-deamino-4′-deoxy-4′-epi-amino-doxorubicin
(2h : $R_1$=H, $R_2$=$NH_2$, $R_3$=OH)

The new anthracycline glycoside antibiotics of the invention, i.e. those of formula 1 and 2, are condensation products of (a) aglycones of general formula 3 or 6 :

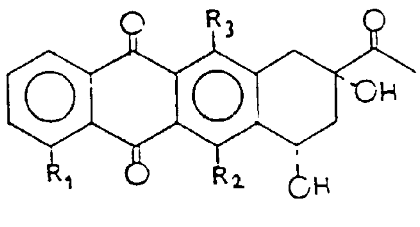

3

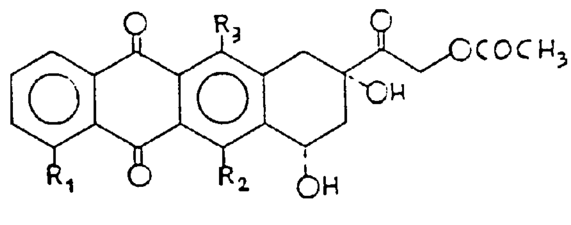

6

wherein $R_1$, $R_2$ and $R_3$ are as defined above with proviso that neither $R_2$ nor $R_3$ is an amino group and (b) a protected halosugar of the formula 4 :

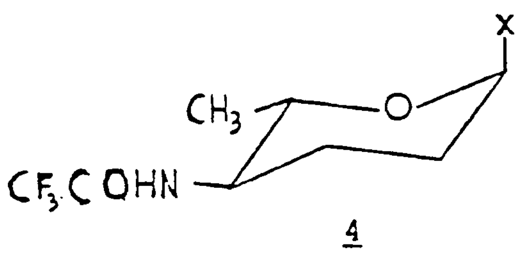

4

wherein X is a halogen, preferably chlorine.

The synthetic methods for the preparation of the above mentioned anthracyclines follows two methods : Method A is depicted in Scheme I and involves the use of the aglycone of formula 3, and Method B, which is shown in Scheme II and involves the use of the aglycone of formula 6. The preparation of the C-6 or C-11 amino compounds of formula 2 is illustrated in Scheme III.

Scheme I

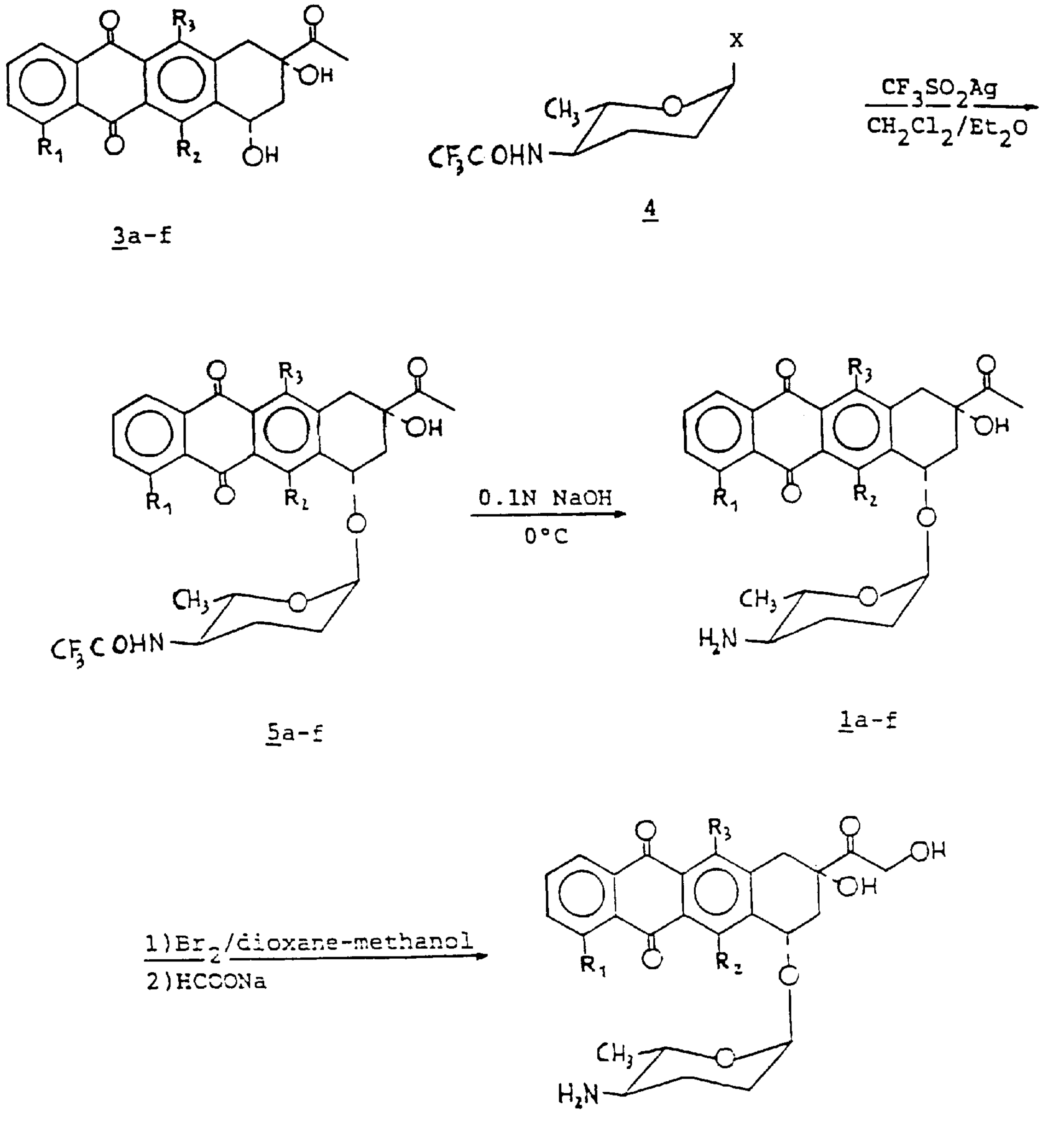


3a-f

4

5a-f

1a-f

2a-f

Scheme II

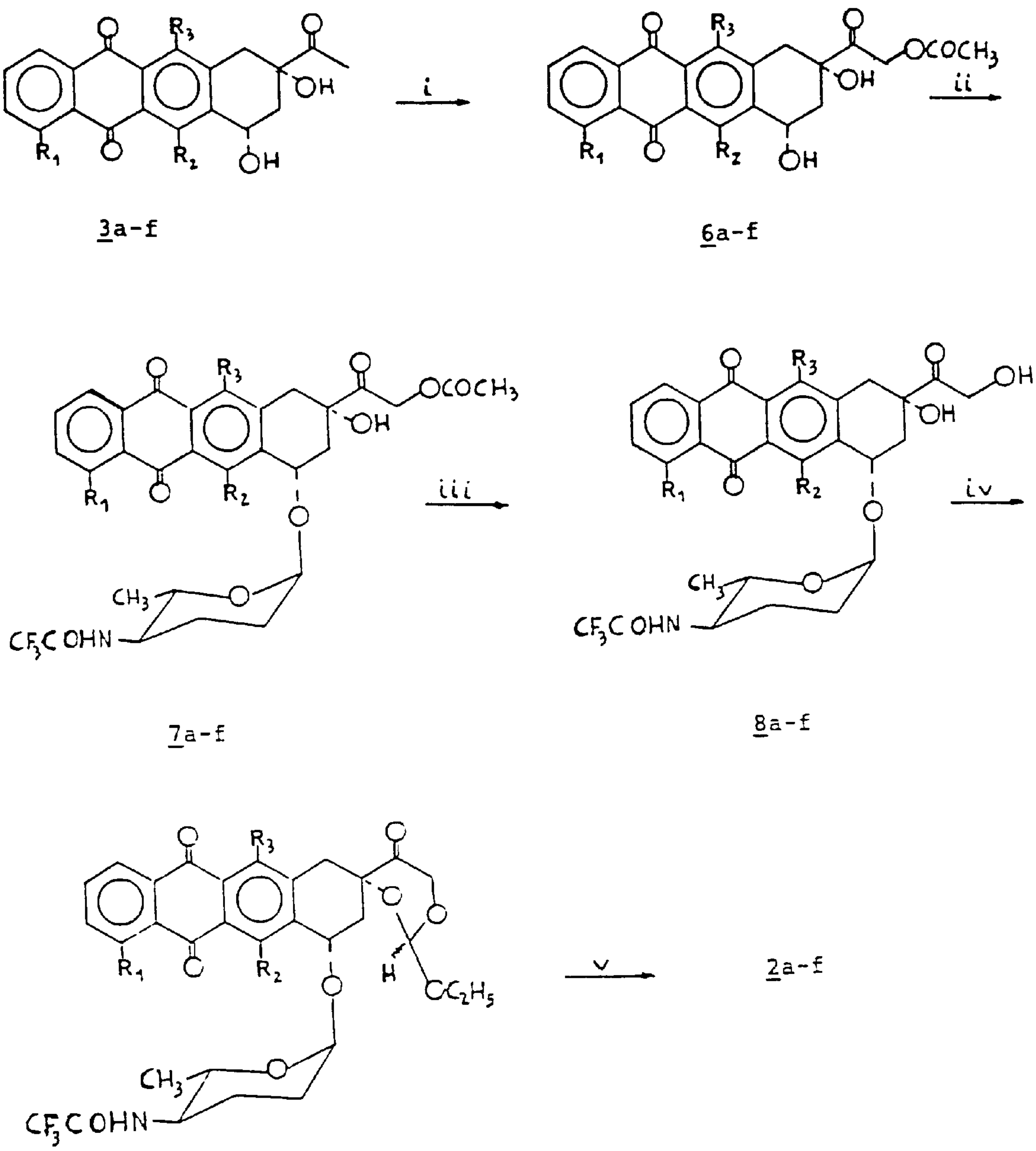


3a-f 6a-f 7a-f 8a-f 9a-f 2a-f

i: $Br_2$/dioxane, $CH_3COOK$; ii: 4, $CF_3SO_2Ag$, $CH_2Cl_2$;
iii: $K_2CO_3$, $CH_3OH$, 0°C; iv: $HC(OC_2H_5)_3$, pTSA-pyridine
v: 0.1N NaOH, $CH_3COOH$

Scheme III

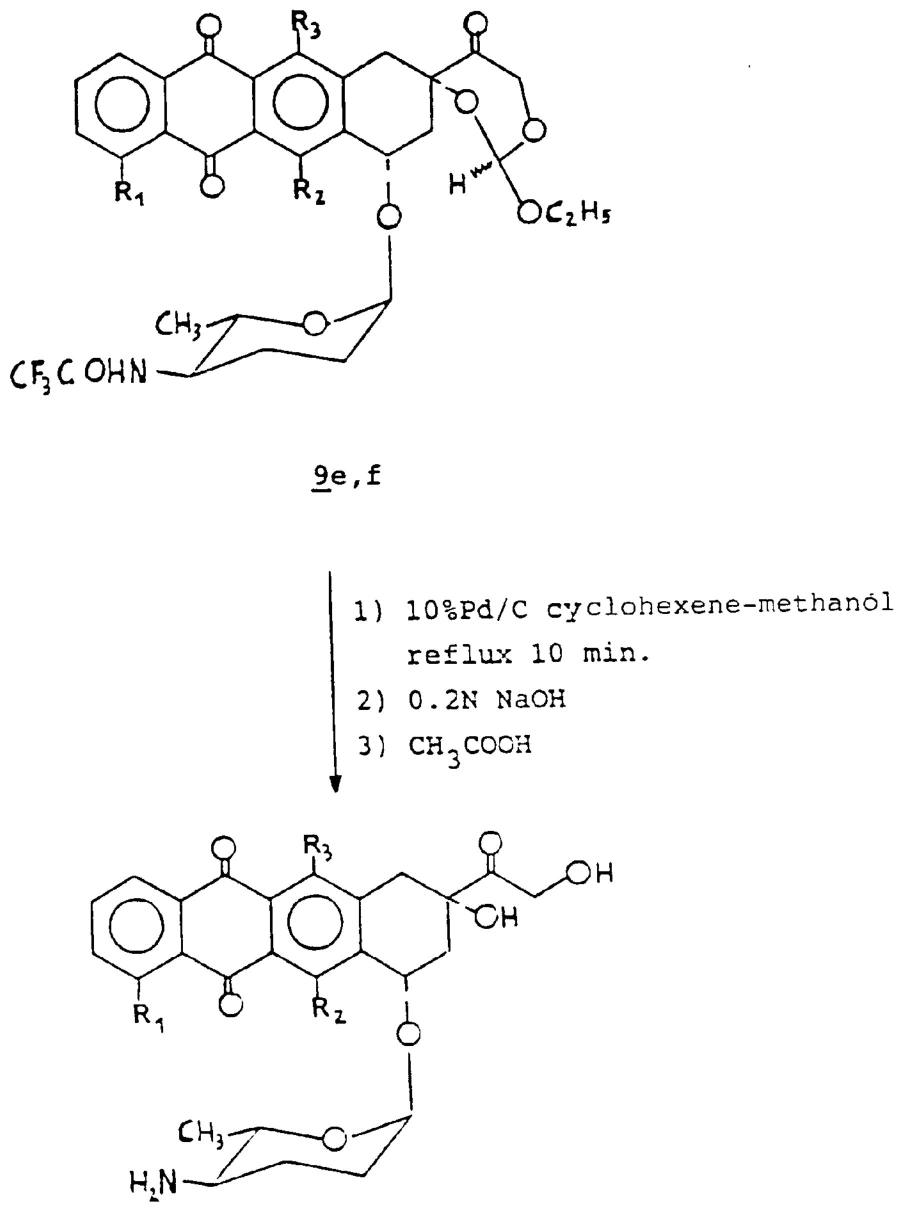


9e,f

2g,h

Method A

The present invention provides a process for the preparation of an anthracycline glycoside of formula 1 or 2 as defined above with proviso that for a glycoside of formula 2 neither $R_2$ nor $R_3$ is an amino group, or a pharmaceutically acceptable acid addition salt thereof, which process comprises :

(i) condensing an aglycone of formula 3 :

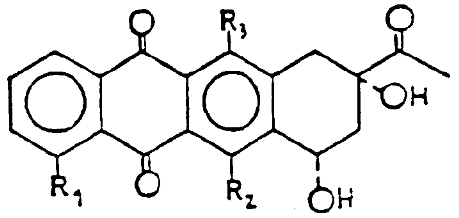

3

wherein $R_1$, $R_2$ and $R_3$ are as defined above except that neither $R_2$ nor $R_3$ is an amino group, with a 1-halo-2,3,4,6-tetradeoxy-4-(N-trifluoroacetamido)-L-erythro-hexopyranoside of formula 4 :

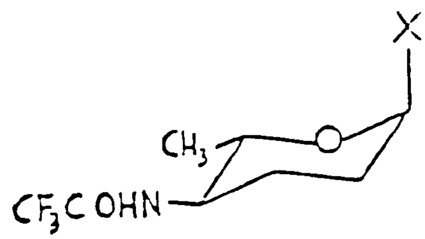

4

wherein X is halogen ;
(ii) removing the N-trifluoroacetyl group from the compound of formula 5 thus obtained :

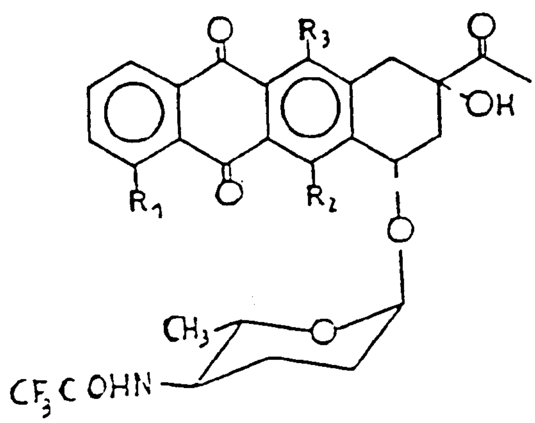

5

wherein $R_1$, $R_2$ and $R_3$ are as defined in step (i), so as to obtain a said anthracycline glycoside of formula 1 except that neither $R_2$ nor $R_3$ is an amino group ;
(iii) if desired, converting the said glycoside of formula 1 obtained in step (ii) into a pharmaceutically acceptable acid addition salt thereof ;
(iv) if desired, reducing a said glycoside of formula 1 wherein one of $R_2$ and $R_3$ is a nitro group obtained in step (ii) or a said salt thereof obtained in step (iii) so as to obtain a said glycoside of formula 1 wherein one of $R_2$ and $R_3$ is an amino group and, if desired, converting the said glycoside of formula 1 wherein one of $R_2$ and $R_3$ is an amino group into a pharmaceutically acceptable acid addition salt thereof ;
(v) if desired, brominating the said glycoside of formula 1 obtained in step (ii) or pharmaceutically acceptable acid addition salt thereof obtained in step (iii) and hydrolysing the 14-bromo derivative thus obtained to form a corresponding anthracycline glycoside of formula 2 as defined above ; and
(vi) if desired, converting the said glycoside of formula 2 into a pharmaceutically acceptable acid addition salt thereof.

This procedure allows the preparation of anthracycline glycosides from the corresponding aglycones 3 and a protected halosugar 4. The procedure is similar to that described in US-A-4,107,423. The coupling product is hydrolyzed to a daunorubicin derivative 1 and can be converted to the corresponding doxorubicin derivative 2 in accordance with the method described in US-A-3,803,124.

The starting materials for the reaction sequences of Scheme I are the well known 4-demethyldaunomycinone (3a : $R_1$=$R_2$=$R_3$=OH), the aglycone 4-demethoxy-4-amino-daunomycinone (3b : $R_1$=$NH_2$, $R_2$=$R_3$=OH) (EP-A-0288268), 4-demethoxy-4-fluorodaunomycinone (3c : $R_1$=F, $R_2$=$R_3$=OH) [G.W. Morrow and J. Swenton, J. Org. Chem. ; 52, 713, 1987] and 4-demethyl-6-deoxydaunomycinone (3d : $R_1$=$R_3$=OH, $R_2$=H) [US-A-4,600,537]. The C-11 and C-6 nitro aglycones 4-demethoxy-11-deoxy-11-nitrodaunomycinone (3e : $R_1$=H, $R_2$=OH, $R_3$=$NO_2$) and 4-demethoxy-6-deoxy-6-nitrodaunomycinone (3f : $R_1$=H, $R_2$=$NO_2$, $R_3$=OH) both described in US-A-4,749,693.

An aglycone of formula 3 is generally reacted at room temperature in step (i) with a compound of formula 4 in the presence of a molecular sieve and silver trifluoromethanesulphonate to form one of the N-trifluoroacetyl glycosides 5a-f. Compounds of formula 1a-f are obtained by removing the amino protecting group by mild alkaline hydrolysis. Preferably in step (ii) the compound of formula 5, dissolved in acetone, is submitted, at a temperature of 0°C and for one hour, to mild alkaline hydrolysis with 0.2 N aqueous sodium hydroxide to give a said glycoside of formula 1. Treatment with methanolic hydrogen chloride yields the hydrochloride.

In step (iv), typically reduction is effected by treatment with Pd/C, for example 10% Pd/C. The nitro glycoside of formula 1 or salt thereof may therefore be refluxed in a mixture of methanol and cyclohexene in the presence of 10% Pd/C for ten minutes. The resulting amino glycoside of formula 1 can be isolated as its hydrochloride as above.

Preferably the said glycoside of formula 1, dissolved in a mixture of anhydrous methanol and dioxane, is treated in step (v) with a chloroform solution of bromine to afford the corresponding 14-bromo derivative which is hydrolyzed for two days at room temperature with an aqueous solution of sodium formate to give a said glycoside of formula 2 as the free base and in step (vi) the said glycoside of formula 2 is isolated as its hydrochloride. Isolation of the glycoside of formula 2 as the hydrochloride is typically achieved by treating the glycoside with methanolic hydrogen chloride.

According to an embodiment of this process 4-demethyldaunomycinone (3a), dissolved in dry methylene chloride, is reacted at room temperature for one hour with 1-chloro-2,3,4,6-tetradeoxy-4-(N-trifluoroacetamido)-L-erythro-hexopyranoside (4) in presence of molecular sieves and silver trifluoromethanesulphonate to obtain the N-protected glycoside 5a which, dissolved in acetone, is submitted at a temperature of 0°C and for one hour, to a mild alkaline hydrolysis with 0.2 N aqueous sodium hydroxide to give the compound of formula 1a as a free base which, by treatment with anhydrous methanolic hydrogen chloride is isolated as its hydrochloride.

If desired, 1a is reacted with bromine in methylene chloride to obtain its 14-bromo derivative from which, after hydrolysis at room temperature and for 48 hours under nitrogen with an aqueous solution of sodium formate, the compound of formula 2a is obtained as free base and, by treatment with anhydrous methanolic hydrogen chloride, is isolated as its hydrochloride.

Method B

The invention provides a process for the preparation of an anthracycline glycoside of formula 2 as defined above, with the proviso that neither $R_2$ nor $R_3$ is an amino group, or a pharmaceutically acceptable acid addition salt thereof, which process comprises :

(i') condensing a 14-protected aglycone of formula 6 :

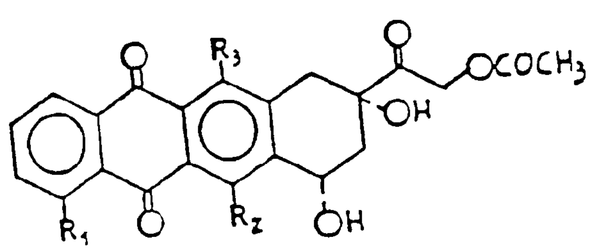

6

wherein $R_1$, $R_2$ and $R_3$ are as defined above except that neither $R_2$ nor $R_3$ is an amino group, with a 1-halo-2,3,4,6-tetradeoxy-4-(N-trifluoroacetamido)-L-erythro-hexopyranoside of formula 4 as defined above ;

(ii') removing the 14-protecting group from the resulting N-protected glycoside of formula 7 :

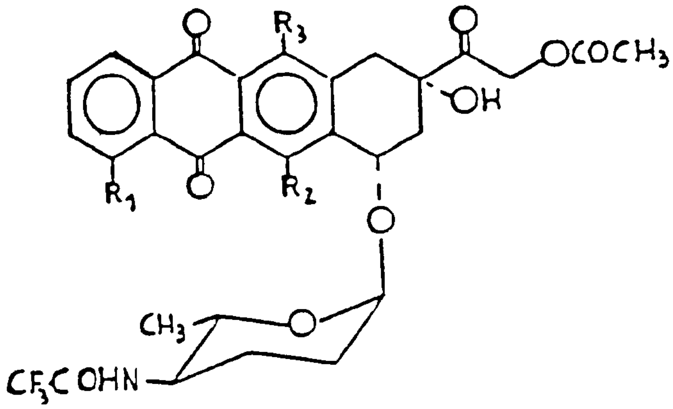

7

wherein $R_1$, $R_2$ and $R_3$ are as defined above, to give the compound of formula 8 :

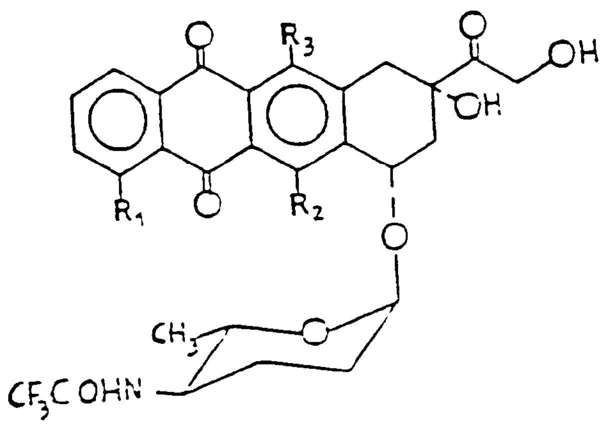

8

wherein $R_1$, $R_2$ and $R_3$ are as defined above ;

(iii') converting the compound of formula 8 into a 9,14-orthoformate derivative of formula 9 :

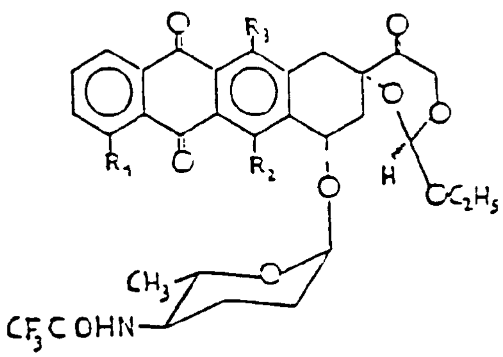

9

wherein $R_1$, $R_2$ and $R_3$ are as defined above ;

(iv') removing the N-trifluoroacetyl group and the orthoformate protecting group to obtain a said glycoside of formula 2 ; and

(v') if desired, converting the said glycoside of formula 2 into a pharmaceutically acceptable acid addition salt thereof.

This procedure allows the preparation of anthracycline glycosides of general formula 2 by coupling (a) the corresponding 14-hydroxylated and protected aglycones and (b) a protected halosugar 4. The procedure is similar to that described in US-A-4,107,423. In Scheme II is depicted the route to doxorubicin derivatives 2a-f. The starting materials are the aglycones 3a-f suitably functionalized and protected in the side chain as 14-acetoxy derivatives.

Typically in step (i′) the aglycone of formula 6 is reacted at room temperature with a 1-chloro-hexapyranoside of formula 4 in the presence of a molecular sieve and silver trifluoromethansulfonate. Preferably in step (ii′) the compound of formula 7 is treated at 0°C, for four hours and under a nitrogen atmosphere with potassium carbonate. Generally in step (iii′) the compound of formula 8 is treated with triethyl orthoformate, for example in methylene chloride and in the presence of pyridinium p-toluensulfonate for one hour at room temperature.

Preferably in step (iv′) the compound of formula 9 is subjected to mild alkaline aqueous hydrolysis to remove the N-trifluoroacetyl group and is treated with acetic acid to remove the orthoformate protecting group to obtain the said glycoside of formula 2 as a free base which, in step (v′), is treated with anhydrous methanolic hydrogen chloride to isolate the glycoside as its hydrochloride salt.

More particularly in one embodiment 4-demethoxy-11-deoxy-11-nitrodaunomycinone (3d : $R_1$=H, $R_2$=OH, $R_3$=$NO_2$) is dissolved in dry dioxane and treated with a solution of bromine in methylene chloride for two hours at room temperature, then precipitated with hexane. The residue, dissolved in acetone, is added with potassium acetate and stirred for one hour to give the acetoxy derivative 6d. This compound is dissolved in dry methylene chloride and reacted with 1-chloro-2,3,4,6-tetradeoxy-4-(N-trifluoroacetamido)-L-erythro-hexopyranoside (4) as described above to give the protected 14-acetoxy-N-trifluoroacetyl glycoside 7d.

In order to remove the protecting groups, compound 7d is first dissolved in methanol and treated with potassium carbonate for four hours at 0°C under nitrogen to give 8d, then is treated with triethyl orthoformate in methylene chloride and in the presence of pyridinium p-toluensulfonate for one hour at room temperature to give the 9,14-orthoformate derivative 9e. This is subjected to mild alkaline aqueous hydrolysis to remove the N-protecting group and finally treated with acetic acid to remove the orthoformate protecting group and give compound 1e as free base which, by treatment with anhydrous methanolic hydrogen chloride, is isolated as its hydrochloride.

The present invention also provides a process for the preparation of an anthracycline glycoside of formula 2 above wherein $R_1$ is as defined above and one of $R_2$ and $R_3$ is hydroxy and the other of $R_2$ and $R_3$ is an amino group, or a pharmaceutically acceptable acid addition salt thereof, which process comprises :

(i″) reducing the C-6 or C-11 nitro group of a 9,14-orthoformate derivative of formula 9 as defined above wherein one of $R_2$ and $R_3$ is hydroxy and the other of $R_2$ and $R_3$ is a nitro group ;

(ii″) removing the N-trifluoroacetyl group and the orthoformate protecting group from the C-6 or C-11 amino group-containing compound thus formed to obtain a said glycoside of formula 2 ; and

(iii″) if desired, converting the said glycoside of formula 2 into a pharmaceutically acceptable acid addition salt thereof.

Scheme III shows the route to C-6 and C-11 amino anthracyclines of formula 2 starting from the corresponding C-6 and C-11 protected nitro glycosides. The 9,14-orthoformate nitro derivatives 9e or 9f may be converted respectively to amino glycosides 2g or 2h by refluxing the nitro compounds in a mixture of methanol and cyclohexene in the presence of 10% Pd/C for ten minutes, and removing the N-trifluoroacetyl group in a basic medium, for example as described above for Method A or Method B, and the orthoformate protecting group with acetic acid. The compounds 2g and 2h may be converted into their respective hydrochloride salts by treatment with anhydrous methanolic hydrogen chloride.

As is apparent from the foregoing, the processes of the invention involve the preparation and use of several novel intermediates. These too are within the scope of the present invention, more especially the compounds of formulae 5 and 7 to 9.

The invention also provides pharmaceutical compositions comprising an anthracycline glycoside of formula 1 or 2 or a pharmaceutically acceptable acid addition salt thereof, together with a pharmaceutically acceptable carrier or diluent. Conventional carriers and diluents may be used. The composition may be formulated and administered in conventional manner.

The compounds of the invention are useful in methods of treatment of the human or animal body by therapy. They are useful as antitumor agents. A therapeutically effective amount is administered to a patient. An amount sufficient to inhibit the growth of the tumour may be administered. The tumour may be a Colon adenocarcinoma or Gross leukemia tumor.

**Biological Assay**

The biological activity of the compounds according to the invention was tested in vitro against LoVo (human colon adenocarcinoma) and LoVo/DX cells in comparison with doxorubicin and 4-demethoxydaunorubicin (4-dem-DNR).

Table 1.

Table 1 - *in vitro* activity

| Compounds | Cytotoxicity after 4h of treatment (IC50[1] = ng/ml) LoVo | LoVo/DX | R.I.[2] |
|---|---|---|---|
| Doxorubicin | 60 | 2180 | 36 |
| 4-dem-DNR | 20 | 125 | 6 |
| 1a | 1.1 | 2.7 | 2.4 |
| 1c | 1.2 | 6.0 | 5.0 |
| 1h | 4.0 | 8.6 | 2.2 |
| 2d | 1.2 | 3.8 | 3.2 |
| 2e | 48.4 | 205 | 4.2 |
| 2f | 116 | 2256 | 19.4 |
| 2g | 18.8 | 109 | 5.8 |
| 2h | 6.2 | 90 | 14.5 |

1) IC50 = concentration inhibiting by 50% colony growth

2) R.I. = Resistance Index = (IC50 LoVo/DX)/(IC50 LoVo)

Example 1

Preparation of 4-demethyl--3′-deamino-4′-deoxy-4′-epi-amino-daunorubicin hydrochloride (1a)

0.76 g (2 mmole) of 4-demethyldaunomycinone (3a) was dissolved with 300 ml of anhydrous methylene chloride in the presence of molecular sieves (4 Å). The mixture was cooled at 10°C, bubbled with nitrogen and added dropwise, under stirring, with 0.86 g (3.2 mmole) of 1-chloro-2,3,4,6-tetradeoxy-4-(N-trifluoroacetamido)-L-*erythro*-hexopyranoside (4) dissolved in 40 ml of anhydrous methylene chloride and 0.78 g (3.0 mmole) of silver trifluoromethanesulphonate dissolved in 40 ml of diethyl ether. After twenty minutes the reaction mixture was treated with 30 ml of aqueous saturated sodium hydrogen carbonate and filtered off. The organic layer was washed with water, dried over anhydrous sodium sulphate and the solvent was removed in vacuo. The residue was chromatographed over a silica gel column using a mixture of methylene chloride acetone (98 : 2 by volume) to give 0.8 g of 4-demethyl-3′-deamino-4′-deoxy-4′-epi-N-trifluoroacetyldaunorubicin (5a). The trifluoroacetyl protecting group was removed by dissolving compound 5a in acetone and treating with aqueous 0.2 N sodium hydroxide at 0°C. After one hour the solution was adjusted to pH 8.1 and extracted repeatedly with methylene chloride. The combined organic extracts, after being dried and concentrated to a small volume were acidified to pH 3.5 with anhydrous methanolic hydrogen chloride. Upon addition of diethyl ether there was obtained 0.64 g (yield 54%) of the title compound 1a as hydrochloride salt. m.p. 168-169°C (with dec.).

TLC on Kieselgel Plate (Merck $F_{254}$) solvent system : methylene chloride/methanol/acetic acid/water (80 : 20 : 7 : 3 by volume) Rf : 0.73. MS-FD : [M] + 497.

$^1$HNMR (200 MHz, DMSO-$d_6$) $\delta$ :

1.21 (d, J=6.2Hz, 3H, 5′-$CH_3$), 1.6-1.8 (m, 4H, 2′-$CH_2$, 3′-$CH_2$), 2.1-2.3 (m, 2H, 8-$CH_2$), 2.27 (s, 3H, $COCH_3$), 2.85 (m, 1H, 4′-H), 2.95 (m, 2H, 10-$CH_2$), 4.11 (dq, J=6.2, 9.9Hz, 1H, 5′-H), 4.98 (m, 1H, 7-H), 5.22 (s, 1H, 9-OH), 7.40 (dd, J=2.0, 7.5Hz, 1H, 3-H), 7.82 (m, 2H, 1-H, 2-H), 8.10 (bs, 3H, 4′-$NH_3$+), 11.96 (s, 1H, 11-OH),

12.87 (s, 1H, 6-OH), 13.40 (s, 1H, 4-OH)

Example 2

Preparation of 4-demethoxy-4-amino-3′-deamino-4′-deoxy-4′-epi-amino-daunorubicin hydrochloride (1b)

0.38 g (1 mmole) of 4-demethoxy-4-aminodaunomycinone (3b) was coupled with 0.37 g (1.5 mmole) of 1-chloro-2,3,4,6-tetradeoxy-4-(N-trifluoroacetamido)-L-erythro-hexopyranoside (4) following the procedure described in Example 1 to afford 0.35 g (yield 60%) of the title compound 1b as hydrochloride salt.

TLC on Kieselgel Plate (Merck $F_{254}$) solvent system : methylene chloride/methanol/acetic acid/water (80 : 20 : 7 : 3 by volume) Rf : 0.45

$^1$HNMR (200 MHz, DMSO-$d_6$) δ (inter alia) :

2.27 (s, 3H, $COCH_3$), 2.85 (m, 1H, 4′-H), 4.98 (m, 1H, 7-H), 6.80 (bd, 2H, 4-$NH_2$), 6.93 (d, J=8.0Hz, 1H, 3-H), 7.46 (t, J=8.0Hz, 1H, 2-H), 7.64 (d, J=8.0Hz, 1H, 1-H), 8.10 (bs, 3H, 4′-$NH_3$+), 13.52 (s, 1H, 11-OH), 14.00 (s, 6-OH)

Example 3

Preparation of 4-demethoxy-4-fluoro-3′-deamino-4′-deoxy-4′-epi-amino-daunorubicin hydrochloride (1c)

0.38 g (1 mmole) of 4-demethoxy-4-fluorodaunomycinone (3c) was coupled with 0.37 g (1.5 mmole) of 1-chloro-2,3,4,6-tetradeoxy-4-(N-trifluoroacetamido)-L-erythro-hexopyranoside (4) following the procedure described in Example 1 to afford 0.48 g of N-trifluoroacetyl derivative 5c.

After removal of the N-protecting group, following standard procedure, was obtained 0.40 g (yield 74%) of the title compound 1c.

TLC on Kieselgel Plate (Merck $F_{254}$) solvent system : methylene chloride/methanol/acetic acid/water (80 : 20 : 7 : 3 by volume) Rf : 0.68, m.p. 158-159°C (with dec.)

Example 4

Preparation of 4-demethyl--3′-deamino-4′-deoxy-4′-epi-amino-doxorubicin hydrochloride (2a)

0.3 g (0.6 mmole) of 4-demethyl-3′-deamino-4′-deoxy-4′-epi-aminodaunorubicin (1a) was dissolved in a mixture of anhydrous methanol and dioxane and added with 1.2 ml of a solution containing 9 g of bromine in 100 ml of methylene chloride, according to the method described in US-A-3,803,124, to afford the 14-bromo derivative, which was dissolved in 20 ml of acetone and treated with 0.4 g of sodium formate dissolved in 2 ml of water. The reaction mixture was stirred at room temperature for two days, then water was added and extracted with methylene chloride. After standard work up, the resulting red solution was concentrated to small volume under vacuum, adjusted to pH 3.5 with anhydrous methanolic hydrogen chloride, then added with an excess of diethyl ether to give 0.2 g, (yield 75%), of the title compound 2a as hydrochloride.

TLC on Kieselgel Plate (Merck $F_{254}$) solvent system : methylene chloride/methanol/acetic acid/water (80 : 20 : 7 : 3 by volume) Rf : 0.60

Example 5

Preparation of 4-demethyl-6-deoxy-14-acetyl-adriamycinone (6d)

To a solution of 0.85 g (2.3 mmole) of 4-demethyl-6-deoxy-daunomycinone (3d), dissolved in 150 ml of anhydrous dioxane, was added 4.6 ml of solution containing 9 g of bromine in 100 ml of methylene chloride. The mixture was left at room temperature for two hours, after that the bromo derivative was precipitated by adding n-hexane and collected. The residue was dissolved with 300 ml of acetone and added under stirring with 2.7 g of potassium acetate. After one hour the reaction mixture was diluted with methylene chloride and washed with water. The organic layer was dried over anhydrous sodium sulphate, filtered off and the solvent was removed under reduced pressure. The residue was chromatographed on a column of silica gel to give 0.75 g (yield 76%) of the title compound 6d.

TLC on Kieselgel Plate (Merck $F_{254}$) solvent system methylene chloride/acetone (95 : 5 by volume) Rf : 0.13

EI-MS : [M] + 426

$^1$HNMR (200 MHz, DMSO $d_6$) δ :

1.88 (dd, J=9.3, 13.1 Hz, 1H, 8ax-H), 2.06 (s, 3H, $COCH_3$), 2.41 (dd, J=5.7, 13.1 Hz, 1H, 8e-H), 2.76-3.04 (two d, J=18.6 Hz, 2H, 10-$CH_2$), 4.57 (m, 1H, 7-H), 5.09-5.23 (two d, J=17.8 Hz, 2H, $COCH_2OCO$), 5.89 (d, J=6.6 Hz, 1H, 7-OH), 6.10 (s, 1H, 9-OH), 7.39 (dd, J=1.5, 7.9Hz, 1H, 3-H), 7.7-7.9 (m, 2H, 1-H, 2-H), 7.90 (s, 1H, 6-H), 12.60-12.90 (two bs, 2H, 4-OH, 11-OH).

Example 6

Preparation of 4-demethyl-6-deoxy-3′-deamino-4′-deoxy-4′-epi-N-trifluoacetyl-doxorubicin (8d)

0.61 g (1.43 mmole) of 4-demethyl-6-deoxy-14-acetyl-adriamycinone (6d) was dissolved with 250 ml of anhydrous methylene chloride and 150 ml of anhydrous tetrahydrofurane. The mixture was cooled at 10°C, bubbled with nitrogen and added dropwise, under stirring, with 1 g (4 mmole) of 1-chloro-2,3,4,6-tetradeoxy-4-(N-trifluoroacetamido)-L-erythro-hexopyranoside (4) dissolved in 50 ml of anhydrous methylene chloride and 1 g (4 mmole) of silver trifluoromethanesulphonate dissolved in 50 ml of diethyl ether. After twenty minutes the reaction mixture was treated with 20 ml of aqueous saturated sodium hydrogen carbonate and filtered off. The organic layer was washed with water, dried over anhydrous sodium sulphate and the solvent was removed in vacuo. The residue was chromatographed over a silica gel column to give 0.4 g (yield 44%) of 4-demethyl-6-deoxy-14-acetyl-3′-deamino-4′-deoxy-4′-epi-N-trifluoroacetyldoxorubicin (7d).

TLC on Kieselgel Plate (Merck $F_{254}$) solvent system methylene chloride/acetone (9 : 1 by volume) RF : 0.45
FD-MS : [M] + 636 $^1$HNMR (200 MHz, DMSO $d_6$) $\delta$, (inter alia) :
1.06 (d, J=6.0 Hz, 3H, 5′-$CH_3$), 1.6-2.0 (m, 5H, 8ax-H, 2′-$CH_2$, 3′-$CH_2$), 2.05 (s, 3H, $COCH_3$) 3.56 (m, 1H, 4′-H), 3.93 (m, 1H, 5′-H), 5.14 (m, 2H, $COCH_2OCO$), 5.17 (m, 1H, 1′-H), 9.37 (bd, J=8.6Hz, 1H, $NHCOCF_3$), 12.45, 12.91 (two s, 2H, 4-OH, 11-OH).

Compound 7d was dissolved with 200 ml of methanol, treated at 0°C with 2 ml of a 10% aqueous solution of potassium carbonate and left to stand for four hours at 0°C under nitrogen.

The solution was neutralized with acetic acid, diluted with water and the product extracted with methylene chloride. After silica gel filtration 0.25 g of the title compound 8d was recovered (yield 89%).

TLC on Kieselgel Plate (Merck $F_{254}$) solvent system : methylene chloride/acetone (9 : 1 by volume) Rf : 0.26.
FD-MS : [M] + 593.

Example 7

Preparation of 4-demethyl-6-deoxy-3′-deamino-4′-deoxy-4′-epi-amino-doxorubicin, hydrochloride (2d)

0.2 g (0.33 mmole) of product 8d, prepared as described in Example 6, was dissolved with 60 ml of dry methylene chloride and added with 15 ml of triethyl orthoformate and 0.1 g of pyridinium p-toluensulfonate. After one hour the solution was washed with water, dried over anhydrous sodium sulphate and reduced to small volume under reduced pressure. The solution was poured into n-hexane and the precipitate of 9,14-orthoformate derivative 9d was collected by filtration.

TLC on Kieselgel Plate (Merck $F_{254}$) solvent system : methylene chloride/acetone (95 : 5 by volume) Rf : 0.7

The residue was dissolved with 200 ml of 0.2 N aqueous sodium hydroxide and the solution was left for ten hours at 8°C under nitrogen. Then the solution was adjusted at pH 8.5 with acetic acid and extracted with methylene chloride. After standard work up, the resulting free base was treated with an aqueous solution of acetic acid for two hours at room temperature. The mixure was brought to pH 8.5 with aqueous sodium hydrogen carbonate and extracted with methylene chloride. The solvent was removed in vacuo and the residue chromatographed on silica gel column to afford 0.12 g (yield 66%) of the title compound as free base which was transformed into the hydrochloride salt 2d by treatment with anhydrous methanolic hydrogen chloride.

TLC on Kieselgel Plate (Merck $F_{254}$) solvent system : methylene chloride/methanol/acetic acid/water (80 : 20 : 7 : 3 by volume) Rf : 0.52. m.p. 155°C (dec.)
FD-MS : [M] + 438
$^1$HNMR (200 MHz, DMSO $d_6$) $\delta$ :
1.21 (d, J=6.2Hz, 3H, 5′-$CH_3$), 1.7-2.0 (m, 5H, 8ax-H, 2′-$CH_2$, 3′-$CH_2$), 2.4-2.5 (m, 1H, 8e-H), 2.77, 3.08 (two d, J=17.7Hz, 2H, 10-$CH_2$), 2.88 (m, 1H, 4′-H), 3.95 (dq, J=6.2, 9.8Hz, 1H, 5′-H), 4.55 (d, J=5.5Hz, 2H, $CH_2OH$), 4.76 (m, 1H, 7-H), 4.84 (t, J=5.5Hz, 1H, $CH_2OH$), 5.20 (m, 1H, 1′-H), 5.93 (s, 1H, 9-OH), 7.40 (dd, J=1.6, 7.7Hz, 1H, 3-H), 7.7-7.9 (m, 2H, 2-H, 1-H), 7.74 (s, 1H, 6-H).

Example 8

Preparation of 4-demethoxy-11-deoxy-11-nitro-14-acetyladriamycinone (6e)

2.3 g (5.7 mmole) of 4-demethoxy-11-deoxy-11-nitro-daunomycinone (3e) was transformed into 1.8 g, (yield 70%), of the title compound 6e following the procedure reported in Example 5. TLC on Kieselgel Plate (Merck $F_{254}$) solvent system : methylene chloride/acetone (95 : 5 by volume) Rf : 0.2.
FD-MS : [M] + 455.
$^1$HNMR (200 MHz, $CDCl_3$) δ :
2.11 (ddd, J=1.8, 5.0, 15.0Hz, 1H, 8ax-H), 2.18 (s, 3H, $COCH_3$), 2.56 (ddd, J=2.2, 2.2, 15.0Hz, 1H, 8e-H), 2.88 (dd, J=2.2, 18.2Hz, 1H, 10e-H), 3.18 (d, J=18.2Hz, 1H, 10ax-H), 3.42 (dd, J=1.8, 3.3Hz. 1H, 7-OH), 4.68 (s, 1H, 9-OH), 5.04, 5.34 (two d, J=18.2Hz, 2H, $COCH_2OCO$), 5.42 (m, 1H, 7-H), 7.88 (m, 2H, 2-H, 3-H), 8.29 (m, 2H, 1-H, 4-H), 13.71 (s, 1H, 6-OH).

Example 9

Preparation of
4-demethoxy-11-deoxy-11-nitro-9,14-orthoformate-3′-deamino-4′-deoxy-4′-epi-N-trifluoroacetyl-doxorubicin (9e)

1.76 g (3.8 mmole) of product 6e was condensed with 1.3 g (5.2 mmole) of the chloro-sugar 4 in presence of silver trifluoromethanesulphonate, following the procedure described in Example 1, to give 1.13 g (yield 44%) of 14-acetyl-N-trifluoroacetyl-derivative 7e.

TLC on Kieselgel Plate (Merck $F_{254}$) solvent system : methylene chloride/acetone (95 : 5 by volume) Rf : 0.37.
FD-MS : [M] + 665 $^1$HNMR (200 MHz, $CDCl_3$) δ :
1.28 (d, J=5.8Hz, 3H, 5′-$CH_3$), 1.8-1.9 (m, 4H, 2′-$CH_2$, 3′-$CH_2$), 2.10 (dd, J=3.8, 15.0Hz, 1H, 8ax-H), 2.18 (s, 3H, $COCH_3$), 2.56 (ddd, J=1.6, 1.6, 15.0Hz, 1H, 8e-H), 2.94 (dd, J=1.6, 18.3Hz, 1H, 10e-H), 3.19 (d, J=18.3Hz, 1H, 10ax-H), 3.7-4.1 (m, 2H, 4′-H, 5′-H), 4.94, 5.32 (two d, J=17.8Hz, 2H, $COCH_2OCO$), 5.40 (m, 2H, 7-H, 1′-H), 6.55 (bd, J=8.6Hz, 1H, $NHC{=}CF_3$), 7.87 (m, 2H, 2-H, 3-H), 8.2-8.4 (m, 2H, 1-H, 4-H), 13.70 (s, 1H, 6-OH)

Product 7e was dissolved with 1000 ml of methanol and, after cooling at 0°C, a 10% aqueous solution of sodium carbonate was added under stirring and nitrogen atmosphere. After three hours, the mixture was brought to pH 7 with acetic acid, diluted with water and the product was extracted with methylene chloride following standard procedure. The crude material was crystallized from diethyl ether to give 0.9 g (yield 87%) of compound 4-demethoxy-11-deoxy-11-nitro-3′-deamino-4′-deoxy-4′-epi-N-trifluoroacetyl-doxorubicin (8e) TLC on Kieselgel Plate (Merck $F_{254}$) solvent system : methylene chloride/acetone (95 : 5 by volume) Rf : 0.17.

Product 8e was treated with triethylorthoformate in presence of pyridinium p-toluensulfonate as described in Example 7 to give 0.78 g (yield 76%) of the title compound 9e.

TLC on Kieselgel Plate (Merck $F_{254}$) solvent system : methylene chloride/acetone (95 : 5 by volume) Rf : 0.48.

Example 10

Preparation of 4-demethoxy-11-deoxy-11-nitro-3′-deamino-4′-deoxy-4′-epi-amino-doxorubicin (2e)

Following the procedure described in Example 7, 0.32 g (0.43 mmole) of compound 9e was first hydrolyzed in basic media to remove the N-protecting group then with acetic acid to remove the orthoformate protecting group. Treatment with anhydrous methanolic hydrogen chloride afforded 0.16 g (yield 61%) of the title compound 2e as hydrochloride with TLC on Kieselgel Plate (Merck $F_{254}$) solvent system : methylene chloride/methanol/acetic acid (80 : 20 : 1 by volume) Rf : 0.30. m.p. 159°C (dec.) MS-FD : [M] + 527
$^1$HNMR (200 MHz, DMSO $d_6$, 50°C) δ :
1.23 (d, J=6.2Hz, 3H, 5′-$CH_3$), 1.7-1.9 (m, 4H, 2′-$CH_2$, 3′-$CH_2$), 2.26 (m, 2H, 8-$CH_2$), 2.82 (m, 2H, 10-$CH_2$), 2.84 (m, 1H, 4′-H), 4.16 (dq, J=6.2, 9.5Hz, 1H, 5′-H), 4.4-4.7 (m, 3H, $COCH_2OH$, $COCH_2OH$), 5.08 (m, 1H, 7-H), 5.24 (m, 1H, 1′-H), 5.53 (s, 1H, 9-OH), 7.98 (m, 2H, 2-H, 3-H), 8.1-8.3 (m, 2H, 1-H, 4-H).

Example 11

Preparation of 4-demethoxy-11-deoxy-11-amino-3′-deamino-4′-deoxy-4′-epi-amino-doxorubicin hydrochloride (2g)

0.32 g (0.43 mmole) of 4-demethoxy-11-deoxy-11-nitro-9,14-ethyl-orthoformate-3′-deamino-4′-deoxy-4′-epi-N-trifluoroacetyldoxorubicin (9e), prepared as described in Example 9, was dissolved in 200 ml of methanol and added with 20 ml of cyclohexene and 0.2 g of 10% Pd/C, under stirring. The mixture was refluxed for ten minutes, then cooled at room temperature and the catalyst was filtered off and the solvent removed under vacuum. The residue was picked up with aqueous 0.2 N sodium hydroxide and kept for eight hours at 10°C under nitrogen. After that, the solution was brought to pH 5 with acetic acid and left to stand for two hours at room temperature. The mixture was neutralized with aqueous sodium hydrogen carbonate and extracted with methylene chloride to give, after standard work up, the 11-amino derivative as free base. Treatment of which with methanolic hydrogen chloride afforded 0.15 g (yield 55%) of the title compound 2g. TLC on Kieselgel Plate (Merck $F_{254}$) solvent system : methylene chloride/methanol/acetic acid (80 : 20 : 1 by volume) Rf : 0.23. m.p. 162-164°C (dec.) FD-MS : [M] + 497
$^1$HNMR (200 MHz, DMSO $d_6$, 50°C) δ :
1.23 (d, J=6.2Hz, 3H, 5′-$CH_3$), 1.7-1.9 (m, 4H, 2′-$CH_2$, 3′-$CH_2$), 2.21 (m, 2H, 8-$CH_2$), 2.80 (m, 2H, 4′-H), 2.88 (m, 2H, 10-$CH_2$), 4.18 (dq, J=6.2, 9.5Hz, 1H, 5′-H), 4.67 (m, 3H, $COCH_2OH$, $COCH_2OH$), 5.05 (m, 1H, 7-H), 5.28 (m, 1H, 1′-H), 5.31 (s, 1H, 9-OH), 7.88 (m, 2H, 2-H, 3-H), 8.27 (m, 2H, 1-H, 4-H), 8.18, 8.40 (two bm, 4H, 11-$NH_2$, 4′-$NH_2$).

Example 12

Preparation of 4-demethoxy-6-deoxy-6-nitro-14-acetyl-adriamycinone (6f)

2 g (5 mmole) of 4-demethoxy-6-deoxy-6-nitro-daunomycinone (3f) was transformed into 1.7 g (yield 75.6%) of 14-acetyl derivative 6f following the procedure described in Example 5. TLC on Kieselgel Plate (Merck $F_{254}$) solvent system : methylene chloride/acetone (95 : 5 by volume) Rf : 0.32. FD-MS : [M] + 455
$^1$HNMR (200 MHz, $CDCl_3$) δ :
2.12 (dd, J=14.9, 4.8Hz, 1H, 8ax-H), 2.20 (s, 3H, $OCOCH_3$), 2.53 (ddd, J=2.0, 2.4, 14.9Hz, 1H, 8e-H), 3.15 (d, J=19.3Hz, 1H, 10ax-H), 3.38 (dd, J=2.0, 19.3Hz, 1H, 10e-H), 3.43 (m, 1H, 7-OH), 5.00 (m, 1H, 7-H), 5.15, 5.31 (two d, J=17.9Hz, 2H, $COCH_2OCO$), 7.89 (m, 2H, 2-H, 3-H), 8.30 (m, 2H, 1-H, 4-H), 14.46 (s, 1H, 11-OH)

Example 13

Preparation of
4-demethoxy-6-deoxy-6-nitro-9,14-orthoformate-3′-deamino-4′-deoxy-4′-epi-N-trifluoroacetyl-doxorubicin (9f)

1.7 g (3.7 mmole) of 4-demethoxy-6-deoxy-6-nitro-14-acetyl-adriamycinone (6f) was coupled with 1.6 g (6 mmole) of 1-chloro-2,3,4,6-tetradeoxy-4-(N-trifluoroacetamido)-L-erythro-hexopyranoside (4) in presence of silver trifluoromethanesulphonate as described in Example 1 to give 1 g (yield 41%) of N-trifluoroacetyl derivative 7f. TLC on Kieselgel Plate (Merck $F_{254}$) solvent system : methylene chloride/acetone (95 : 5 by volume) Rf : 0.50.
FD-MS : [M] + 665

0.5 g (0.75 mmole) of compound 7f was treated with triethylorthoformate in presence of pyridinium p-toluensulfonate as described in Example 9 to afford, after chromatographic separation, 0.3 g (yield 60%) of the title compound 9f as mixture of two diastereoisomers with a molar ratio of 80/20. TLC on Kieselgel Plate (Merck $F_{254}$) solvent system : methylene chloride/acetone (95 : 5 by volume) Rf : 0.65.
$^1$HNMR (220 MHz, $CDCl_3$) δ :
1.1-1.3 (m, 6H, 5′-$CH_3$, $OCH_2CH_3$), 1.6-2.0 (m, 4H, 2′-$CH_2$, 3′-$CH_2$), 2.3-2.7 (m, 2H, 8-$CH_2$), 3.33 (s, 2H, 10--$CH_2$), 3.6-3.7 (m, 2H, $OCH_2CH_3$), 3.75 (m, 1H, 4′-H), 3.90 (dq, J=6.2, 9.5Hz, 1H, 5′-H), 4.32, 4.45 (two d, J=17.4Hz, $COCH_2O$ major isomer), 4.20, 4.35 (two d, J=16.8Hz, $COCH_2O$ minor isomer), 4.87 (m, 1H, 1′-H), 5.01, 5.14 (dd, J=5.3, 5.3Hz, 1H, 7-H major, 7-H minor), 5.70, 5.72 (s, 1H, $CH$-$OCH_2CH_3$ major, $CH$-$OCH_2CH_3$ minor), 6.04 (bd, J=9.0Hz, 1H, $NHCOCF_3$), 7.85 (m, 2H, 2-H, 3-H), 8.27 (m, 2H, 1-H, 4-H), 13.52, 13.55 (s, 1H, 11-OH major, 11-OH minor)

Example 14

Preparation of 4-demethoxy-6-deoxy-6-nitro-3′-deamino-4′-deoxy-4′-epi-amino doxorubicin hydrochloride (2f)

0.3 g (0.44 mmole) of compound 9f, prepared as described above, was dissolved in 200 ml of 0.2 N aqueous sodium hydroxide and kept for ten hours at 0°C under nitrogen. The solution was brought at pH 8.5 with acetic acid and extracted with methylene chloride. The aqueous solution was adjusted to pH 8.1 and extracted repeatedly with methylene chloride. The combined organic extracts, after being dried and concentrated to a small volume were acidified to pH 3.5 with anhydrous methanolic hydrogen chloride.

Upon addition of diethyl ether there was obtained 0.062 g (yield 25%) of the title compound 2f as hydrochloride salt. TLC on Kieselgel Plate (Merck $F_{254}$) solvent system : methylene chloride/methanol/acetic acid/water (30 : 4 : 1 : 0.5 by volume) Rf : 0.30 m.p. 155-157°C (with dec.) MS-FD : [M] + 527

$^1$HNMR (200 MHz, DMSO $d_6$) δ :
1.18 (d, J=6.3Hz, 3H, 5′-$CH_3$), 1.6-1.9 (m, 4H, 2′-$CH_2$, 3′-$CH_2$), 2.1-2.5 (m, 2H, 8-$CH_2$), 2.84 (m, 1H, 4′-H), 3.01 (m, 2H, 10-$CH_2$), 4.06 (dq, J=6.3, 9.3Hz, 1H, 5-H), 4.53 (m, 2H, $CH_2OH$), 5.07 (m, 1H, 7-H), 5.26 (m, 1H, 1′-H), 5.50 (m, 1H, $CH_2OH$), 7.88 (m, 2H, 2-H, 3-H), 8.14 (bs, 3H, 4′-$NH_3$+), 8.24 (m, 2H, 1-H), 4-H)

Example 15

Preparation of 4-demethoxy-6-deoxy-6-amino-3′-deamino-4′-deoxy-4′-epi-amino-doxorubicin hydrochloride (2h)

0.15 g (0.22 mmole) of 9,14-orthoformate derivative 9f, prepared as described in Example 13, was dissolved in 150 ml of methanol and 15 ml of cyclohexene and treated with 0.15 g 10% Pd/C as described in Example 11 to give, upon addition with anhydrous methanolic hydrogen chloride, 0.02 g (yield 18.5%) of the 6-amino derivative 2h.

TLC on Kieselgel Plate (Merck $F_{254}$) solvent system : methylene chloride/methanol/acetic acid/water (30 : 4 : 1 : 0.5 by volume) Rf : 0.22. MS-FD : [M] + 497

$^1$HNMR (200 MHz, DMSO $d_6$, 50°C) δ :
1.18 (d, J=6.3Hz, 3H, 5′-$CH_3$), 1.6-1.9 (m, 4H, 2′-$CH_2$, 3′$CH_2$), 2.1-2.5 (m, 2H, 8-$CH_2$), 2.84 (m, 1H, 4′-H), 3.01 (m, 2H, 10-$CH_2$), 4.06 (bq, J=6.3, 9.3Hz, 1H, 5-H), 4.53 (m, 2H, $CH_2OH$), 5.07 (m, 1H, 7-H), 5.26 (1H, 1′-H), 5.50 (m, 1H, $CH_2OH$), 7.88 (m, 2H, 2-H, 3-H), 8.14 (bs, 3H, 4′-$NH_3$+), 8.24 (m, 2H, 1-H, 4-H)

Example 16

Preparation of 4-demethoxy-6-deoxy-6-nitro-3′-deamino-4′-deoxy-4′-epi-amino daunorubicin hydrochloride (1f)

0.78 g (1.76 mmole) of 4-demethoxy-6-deoxy-6-nitrodaunomycinone (3f) was coupled with 1-chloro-2,3,4,6-tetradeoxy-4-(N-trifluoroacetamido)-L-erythro-hexopyranoside (4) following the procedure described in Example 1 to afford 0.26 g (yield 62%) of the title compound 1f as hydrochloride salt.

TLC on Kieselgel plates (Merck $F_{254}$) solvent system : methylene chloride/methanol/acetic acid/water (80: 20 : 7 : 3 by volume) Rf = 0.42. MS-FD [M] + 511

$^1$H NMR (200 MHz, DMSO-$d_6$) inter alia δ :
2.34 (s, 3H, $COCH_3$) ; 3.10 (d, J=18.7 Hz, 1H, 10ax-H) ; 3.27 (dd, J=1.8, 18.7Hz, 1H 10eq-H), 5.11 (dd, J=2.3, 4.3Hz, 1H, 7-H) ; 7.8-7.9 (m, 2H, 2-H, 3-H) ; 8.10 (bs, 3H, 4′-$NH_3$+) ; 8.2-8.4 (m, 2H, 1-H, 4-H) ; 13.55 (s, 1H, 11-OH).

Example 17

Preparation of 4-demethoxy-6-deoxy-6-amino-3′-deamino-4′-deoxy-4′-epi-aminodaunorubicin hydrochloride (1h)

0.3 g (0.5 mmole) of compound 1f was dissolved in 200 ml of methanol and 20 ml of cyclohexene and treated with 0.2 g of 10% Pd/C. After refluxing for ten minutes, the catalyst was filtered off and solvent removed in vacuo. After crystallisation from methanol/ethyl ether, 0.2 g (Yield 76%) of the title compound 1h, as hydrochloride salt, was obtained. TLC on Kieselgel plates (Merck $F_{254}$) solvent system ; methylene chloride/methanol/acetic

acid/water (80 : 20 : 7 : 3 by volume) Rf=0.39. MS-FD : [M] + 431
$^1$H NMR (200 MHz, DMSO-$d_6$) inter alia δ :
1.18 (d, J=6.3 Hz, 3H, 5′-$CH_3$) ; 1.6-1.9 (m, 4H, 2′-$CH_2$, 3′-$CH_2$) ; 2.1-2.5 (m, 2H, 8-$CH_2$) ; 2.84 (m, 1H, 4′-H) ; 3.01 (m, 2H, 10-$CH_2$) ; 4.06 (bq, J=6.3, 9.3Hz, 1H, 5-H) ; 5.7 (m, 1H, 7-H) ; 5.26 (m, 1H, 1′-H) ; 7.8-7.9 (m, 2H, 2-H, 3-H) ; 8.10 (bs, 3H, 4′-$NH_3$+) ; 8.2-8.4 (m, 2H, 1-H,4-H).

Example 18

Preparation of 4-demethoxy-11-deoxy-11-nitro-3′-deamino-4′-deoxy-4′-epi-amino-daunorubicin hydrochloride (1e)

0.58 g (1.46 mmole) of 4-demethoxy-11-deoxy-11-nitrodaunomycinone (3e) was coupled with sugar (4) following the procedure described in Example 1 to afford, after usual work up, 0.5 g (yield 61%) of the title compound 1e as hydrochloride salt.

TLC on Kieselgel plates (Merck $F_{254}$) solvent system : methylene chloride/methanol/acetic acid/water (80: 20 : 7 : 3 by volume) Rf = 0.44. MS-FD [M] + 511
$^1$H NMR (200 MHz, DMSO-$d_6$) inter alia δ :
1.18 (d, J=6.3Hz, 3H, 5′-$CH_3$) ; 1.6-1.8 (m, 4H, 2′-$CH_2$, 3′-$CH_2$) ; 2.1-2.5 (m, 2H, 8-$CH_2$) ; 2.37 (s, 3H, $COCH_3$); 3.07 (m, 2H, 10-$CH_2$) ; 4.06 (bq, J=6.3, 9.3Hz, 1H, 5′-H) ; 5.03 (m, 1H, 7-H) ; 5.32 (m, 1H, 1′-H) ; 7.8-7.9 (m, 2H, 2-H, 3-H) ; 8.10, bs, 3H, 4-$NH_3$+) ; 8.2-8.4 (m, 2H, 1H, 4H), 13.7 (s, 1H, 6-OH).

Example 19

Preparation of 4-demethoxy-11-deoxy-11-amino-3′-deamino-4′-deoxy-4′-epiaminodaunorubicin hydrochloride (1g)

0.2 g (0.36 mmole) of compound 1e was transformed into its reduced amino derivative 1g, following the procedure described in Example 17.

Yield 80%.

TLC on Kieselgel plates (Merck $F_{254}$) solvent system : methylene chloride/methanol/acetic acid/water (80 : 20: 7 : 3 by volume) Rf = 0.41. MS-FD [M] + 481.

## Claims

### Claims for the following Contracting States : AT, BE, CH, DE, DK, FR, GB, IT, LI, NL, SE

1. An anthracycline glycoside of general formula 1 or 2 :

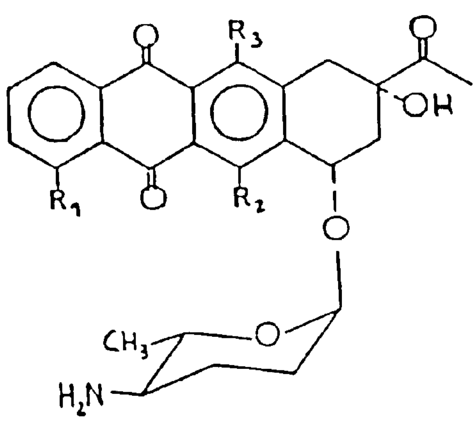

1

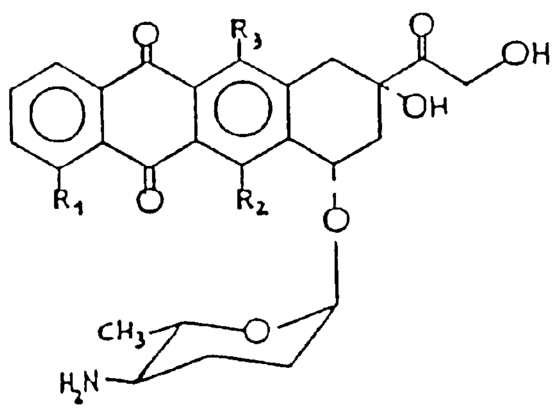

2

wherein $R_1$ is selected from the group consisting of hydrogen, fluorine, hydroxy or amino ; $R_2$ and $R_3$ both represent hydroxy or one of $R_2$ and $R_3$ is hydrogen, nitro or amino and the other of $R_2$ and $R_3$ is hydroxy ; with the

provise that when $R_2$ and $R_3$ are both hydroxy, the $R_1$ is fluorine hydroxy or amino ; and pharmaceutically acceptable acid addition salts thereof.

2. A compound according to claim 1, which is the anthracycline glycoside of formula 1 4-demethyl-3'-deamino-4'-deoxy-4'-epi-amino-daunorubicin or its hydrochloride.

3. A compound according to claim 1, which is the anthracycline glycoside of formula 1 4-demethoxy-4-amino-3'-deamino-4'-deoxy-4'-epi-amino-daunorubicin or its hydrochloride.

4. A compound according to claim 1, which is the anthracycline glycoside of formula 1 4-demethoxy-4-fluoro-3'-deamino-4'-deoxy-4'-epi-amino daunorubicin or its hydrochloride.

5. A compound according to claim 1, which is the anthracycline glycoside of formula 2 4-demethyl-3'-deamino-4'-deoxy-4'-epi-amino-doxorubicin or its hydrochloride.

6. A compound according to claim 1, which is the anthracycline glycoside of formula 2 4-demethyl-6-deoxy-3'-deamino-4'-deoxy-4'-epi-amino-doxorubicin or its hydrochloride.

7. A compound according to claim 1, which is the anthracycline glycoside of formula 2 4-demethoxy-11-deoxy-11-nitro-3'-deamino-4'-deoxy-4'-epi-amino-doxorubicin or its hydrochloride.

8. A compound according to claim 1, which is the anthracycline glycoside of formula 2 4-demethoxy-11-deoxy-11-amino-3'-deamino-4'-deoxy-4'-epi-amino-doxorubicin or its hydrochloride.

9. A compound according to claim 1, which is the anthracycline glycoside of formula 2 4-demethoxy-6-deoxy-6-nitro-3'-deamino-4'-deoxy-4'-epi-amino-doxorubicin or its hydrochloride.

10. A compound according to claim 1, which is the anthracycline glycoside of formula 2 4-demethoxy-6-deoxy-6-amino-3'-deamino-4'-deoxy-4'-epi-amino-doxorubicin or its hydrochloride.

11. A process for the preparation of an anthracycline glycoside of formula 1 or 2 as defined in claim 1 with proviso that for a glycoside of formula 2 neither $R_2$ nor $R_3$ is an amino group, or a pharmaceutically acceptable acid addition salt thereof, which process comprises :

(i) condensing an aglycone of formula 3 :

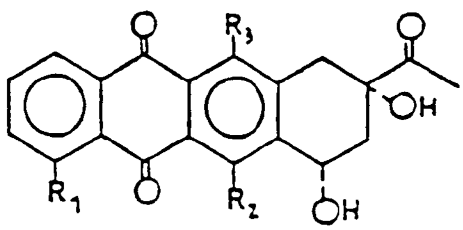

3

wherein $R_1$, $R_2$ and $R_3$ are as defined in claim 1 except that neither $R_2$ nor $R_3$ is an amino group, with a 1-halo-2,3,4,6-tetradeoxy-4-(N-trifluoroacetamido)-L-erythro-hexopyranoside of formula 4 :

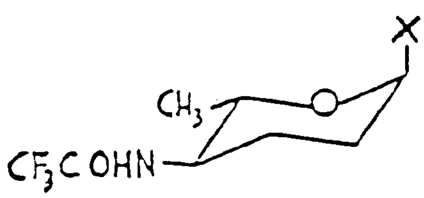

4

wherein X is halogen ;

(ii) removing the N-trifluoroacetyl group from the compound of formula 5 thus obtained :

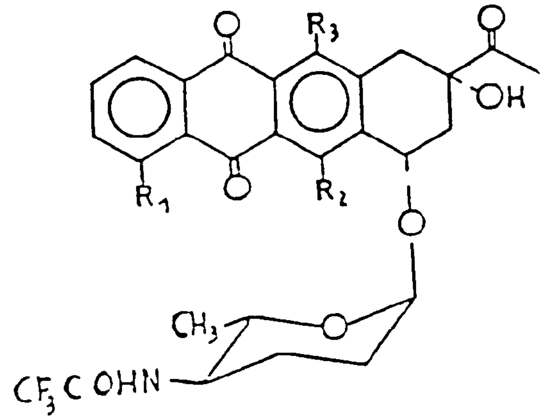

5

wherein $R_1$, $R_2$ and $R_3$ are as defined in step (i), so as to obtain a said anthracycline glycoside of formula 1 except that neither $R_2$ nor $R_3$ is an amino group ;

(iii) if desired, converting the said glycoside of formula 1 obtained in step (ii) into a pharmaceutically acceptable acid addition salt thereof ;

(iv) if desired, reducing a said glycoside of formula 1 wherein one of $R_2$ and $R_3$ is a nitro group obtained in step (ii) or a said salt thereof obtained in step (iii) so as to obtain a said glycoside of formula 1 wherein one of $R_2$ and $R_3$ is an amino group and, if desired, converting the said glycoside of formula 1 wherein one of $R_2$ and $R_3$ is an amino group into a pharmaceutically acceptable acid addition salt thereof ;

(v) if desired, brominating the said glycoside of formula 1 obtained in step (ii) or pharmaceutically acceptable acid addition salt thereof obtained in step (iii) and hydrolysing the 14-bromo derivative thus obtained to form a corresponding anthracycline glycoside of formula 2 as defined above ; and

(vi) if desired, converting the said glycoside of formula 2 into a pharmaceutically acceptable acid addition salt thereof.

12. A process according to claim 11, wherein in step (i) the aglycone of formula 3 is reacted at room temperature with a 1-chloro-hexopyranoside of formula 4 in the presence of a molecular sieve and silver trifluoromethanesulfonate.

13. A process according to claim 11 or 12, wherein in step (ii) the compound of formula 5, dissolved in acetone, is submitted, at a temperature of 0°C and for one hour, to mild alkaline hydrolysis with 0.2 N aqueous sodium hydroxide to give a said glycoside of formula 1.

14. A process according to any one of claims 11 to 13, wherein the said glycoside of formula 1, dissolved in a mixture of anhydrous methanol and dioxane, is treated in step (v) with a chloroform solution of bromine to afford the corresponding 14-bromo derivative which is hydrolyzed for two days at room temperature with an aqueous solution of sodium formate to give a said glycoside of formula 2 as the free base and in step (vi) the said glycoside of formula 2 is isolated as its hydrochloride.

15. A process for the preparation of an anthracycline glycoside of formula 2 as defined in claim 1, with the proviso that neither $R_2$ nor $R_3$ is an amino group, or a pharmaceutically acceptable acid addition salt thereof, which process comprises :

(i') condensing a 14-protected aglycone of formula 6 :

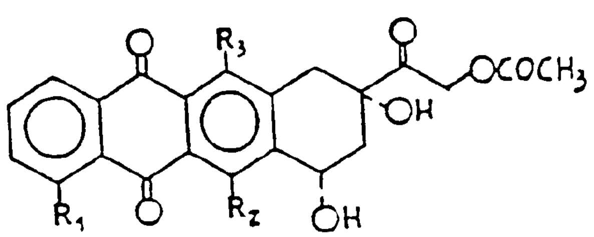

6

wherein $R_1$, $R_2$ and $R_3$ are as defined in claim 1 except that neither $R_2$ nor $R_3$ is an amino group, with a 1-halo-2,3,4,6-tetradeoxy-4-(N-trifluoroacetamido)-L-erythro-hexopyranoside of formula 4 as defined in claim 11 ;

(ii') removing the 14-protecting group from the resulting N-protected glycoside of formula 7 :

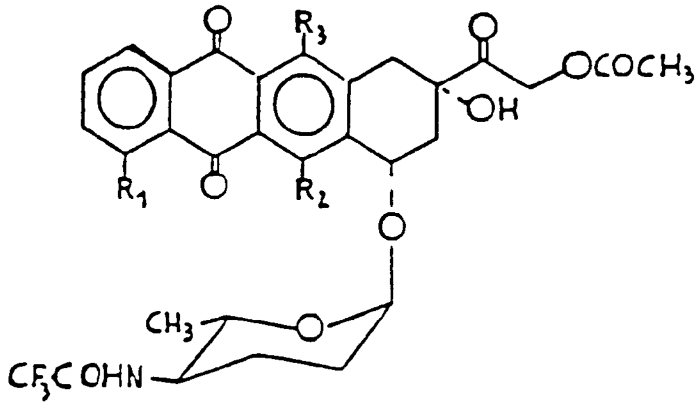

7

wherein $R_1$, $R_2$ and $R_3$ are as defined above, to give the compound of formula 8 :

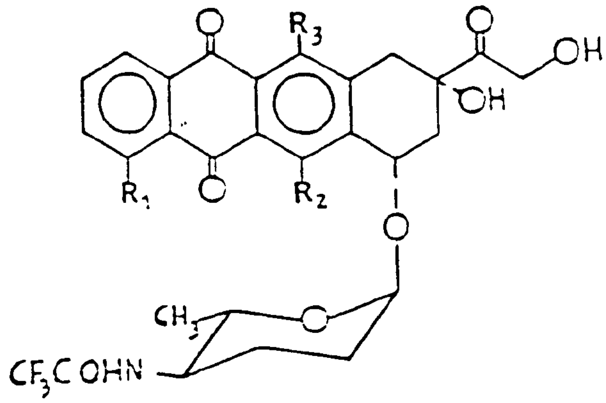

8

wherein $R_1$, $R_2$ and $R_3$ are as defined above ;

(iii') converting the compound of formula 8 into a 9,14-orthoformate derivative of formula 9 :

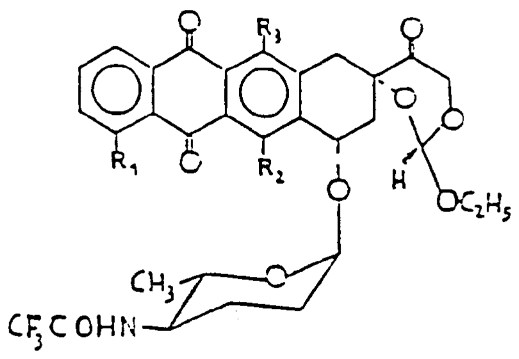

9

wherein $R_1$, $R_2$ and $R_3$ are as defined above ;

(iv') removing the N-trifluoroacetyl group and the orthoformate protecting group to obtain a said glycoside of formula 2 ; and

(v') if desired, converting the said glycoside of formula 2 into a pharmaceutically acceptable acid addition salt thereof.

16. A process according to claim 15, wherein in step (i') the aglycone of formula 6 is reacted at room temperature with a 1-chloro-hexapyranoside of formula 4 in the presence of a molecular sieve and silver trifluoromethansulfonate.

17. A process according to claim 15 or 16, wherein in step (ii') the compound of formula 7 is treated at 0°C, for four hours and under a nitrogen atmosphere with potassium carbonate.

18. A process according to any one of claims 15 to 17, wherein in step (iii') the compound of formula 8 is

treated with triethyl orthoformate.

19. A process according to claim 18, wherein the compound of formula 8 is treated with the triethyl orthoformate in methylene chloride and in the presence of pyridinium p-toluensulfonate for one hour at room temperature.

20. A process according to any one of claims 15 to 19, wherein in step (iv′) the compound of formula 9 is subjected to mild alkaline aqueous hydrolysis to remove the N-trifluoroacetyl group and is treated with acetic acid to remove the orthoformate protecting group to obtain the said glycoside of formula 2 as a free base which, in step (v′), is treated with anhydrous methanolic hydrogen chloride to isolate the glycoside as its hydrochloride salt.

21. A process for the preparation of an anthracycline glycoside of formula 2 according to claim 1 wherein $R_1$ is as defined in claim 1 and one of $R_2$ and $R_3$ is hydroxy and the other of $R_2$ and $R_3$ is an amino group, or a pharmaceutically acceptable acid addition salt thereof, which process comprises :

(i″) reducing the C-6 or C-11 nitro group of a 9,14-orthoformate derivative of formula 9 as defined in claim 15 wherein one of $R_2$ and $R_3$ is hydroxy and the other of $R_2$ and $R_3$ is a nitro group ;

(ii″) removing the N-trifluoroacetyl group and the orthoformate protecting group from the C-6 or C-11 amino group-containing compound thus formed to obtain a said glycoside of formula 2 ; and

(iii″) if desired, converting the said glycoside of formula 2 into a pharmaceutically acceptable acid addition salt thereof.

22. A process according to claim 21, wherein step (i″) is effected by refluxing the 9,14-orthoformate derivative of formula 9 in a mixture of methanol and cyclohexene in the presence of 10% Pd/C for ten minutes.

23. A process according to claim 21 or 22, wherein the N-trifluoroacetyl group is removed in step (ii″) in a basic medium and the orthoformate protecting group is removed with acetic acid to give a said glycoside of formula 2 which is converted in step (iii″) by treatment with anhydrous methanolic hydrogen chloride into its hydrochloride.

24. A pharmaceutical composition comprising an anthracycline glycoside of formula 1 and 2 as defined in claim 1, or a pharmaceutically acceptable acid addition salt thereof, together with a pharmaceutically acceptable carrier or diluent.

25. An anthracycline glycoside of formula 1 and 2, as defined in claim 1, or a pharmaceutically acceptable acid addition salt thereof, for use in a method of treatment of the human or animal body by therapy.

26. A compound according to claim 25 for use as an antitumor agent.

27. A compound of formula 5 as defined in claim 11.

28. A compound of formula 7, 8 or 9 as defined in claim 15.

**Claims for the following Contracting States : ES, GR**

1. A process for the preparation of an anthracyline glycoside of formula 1 or 2

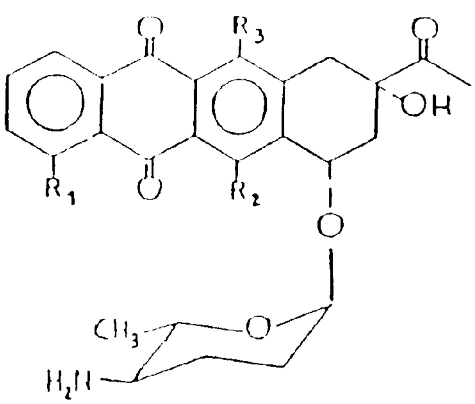

1

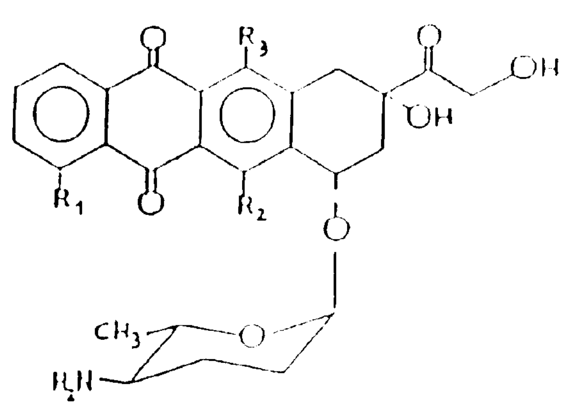

2

wherein $R_1$ is selected from the group consisting of hydrogen, fluorine, hydroxy and amino ; $R_2$ and $R_3$ both represent hydroxy or one of $R_2$ and $R_3$ is hydrogen, nitro or amino and the other of $R_2$ and $R_3$ is hydroxy ; with the proviso that when $R_2$ and $R_3$ are both hydroxy, then $R_1$ is fluorine, hydroxy or amino ; and pharmaceutically

acceptable acid additions salts thereof, which process comprises :

(i) condensing an aglycone of formula 3 :

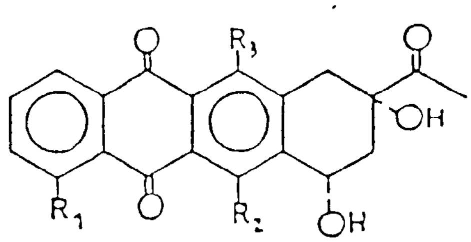

3

wherein $R_1$, $R_2$ and $R_3$ are as defined above except that neither $R_2$ nor $R_3$ is an amino group, with a 1-halo-2,3,4,6,-tetradeoxy-4-(N-trifluoroacetamido)-L-erythro-hexopyranoside of formula 4.

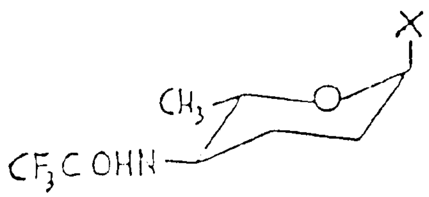

4

wherein X is halogen ;

(ii) removing the N-trifluoroacetyl group from the compound of formula 5 thus obtained :

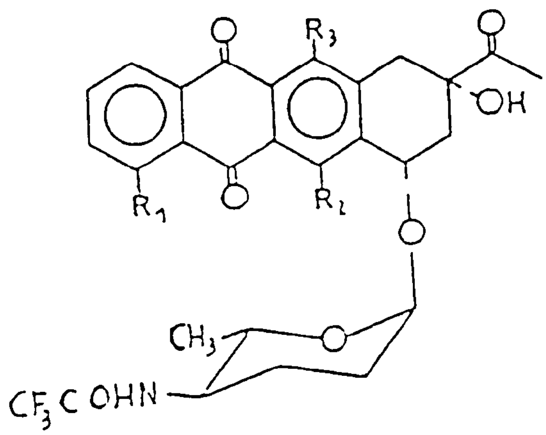

5

wherein $R_1$, $R_2$ and $R_3$ are as defined in step (i), so as to obtain a said anthracycline glycoside of formula 1 except that neither $R_2$ nor $R_3$ is an amino group ;

(iii) if desired, converting the said glycoside of formula 1 obtained in step (ii) into a pharmaceutically acceptable acid addition salt thereof ;

(iv) if desired, reducing a said glycoside of formula 1 wherein one of $R_2$ and $R_3$ is a nitro group obtained in step (ii) or a said salt thereof obtained in step (iii) so as to obtain a said glycoside of formula 1 wherein one of $R_2$ and $R_3$ is an amino group and, if desired, converting the said glycoside of formula 1 wherein one of $R_2$ and $R_3$ is an amino group into a pharmaceutically acceptable acid addition salt thereof ;

(v) if desired, brominating the said glycoside of formula 1 obtained in step (ii) or pharmaceutically acceptable acid addition salt thereof obtained in step (iii) and hydrolysing the 14-bromo derivative thus obtained to form a corresponding anthracycline glycoside of formula 2 as defined above ; and

(vi) if desired, converting the said glycoside of formula 2 into a pharmaceutically acceptable acid addition salt thereof.

2. A process according to claim 1, wherein in step (i) the aglycone of formula 3 is reacted at room temperature with a 1-chloro-hexopyranoside of formula 4 in the presence of a molecular sieve and silver trifluoromethanesulfonate.

3. A process according to claim 1 or 2, wherein in step (ii) the compound of formula 5, dissolved in acetone, is submitted, at a temperature of 0°C and for one hour, to mild alkaline hydrolysis with 0.2 N aqueous sodium hydroxide to give a said glycoside of formula 1.

4. A process according to any one of claims 1 to 3, wherein the said glycoside of formula 1, dissolved in a mixture of anhydrous methanol and dioxane, is treated in step (v) with a chloroform solution of bromine to afford the corresponding 14-bromo derivative which is hydrolyzed for two days at room temperature with an aqueous solution of sodium formate to give a said glycoside of formula 2 as the free base and in step (vi) the said glycoside of formula 2 is isolated as its hydrochloride.

5. A process for the preparation of an anthracycline glycoside of formula 2 as defined in claim 1, with the proviso that neither $R_2$ nor $R_3$ is an amino group, or a pharmaceutically acceptable acid addition salt thereof, which process comprises :

(i') condensing a 14-protected aglycone of formula 6 :

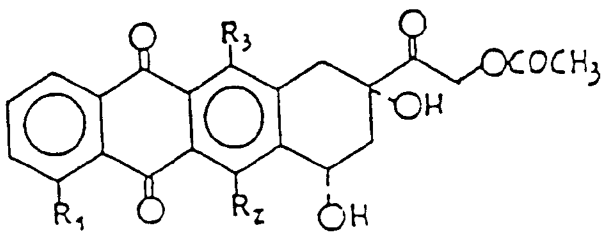

6

wherein $R_1$, $R_2$ and $R_3$ are as defined in claim 1 except that neither $R_2$ nor $R_3$ is an amino group, with a 1-halo-2,3,4,6-tetradeoxy-4-(N-trifluoroacetamido)-L-erythro-hexopyranoside of formula 4 as defined in claim 11 ;

(ii') removing the 14-protecting group from the resulting N-protected glycoside of formula 7 :

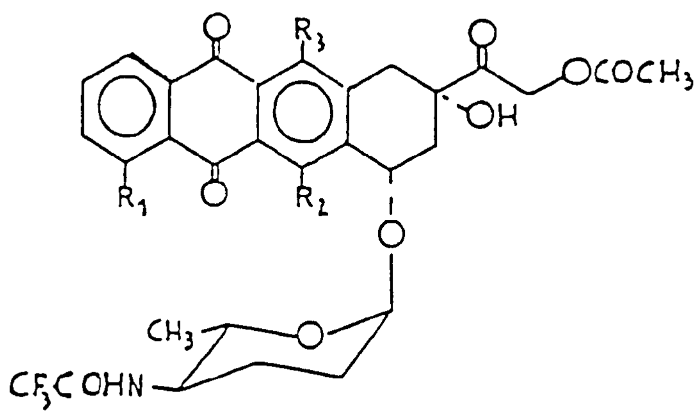

7

wherein $R_1$, $R_2$ and $R_3$ are as defined above, to give the compound of formula 8 :

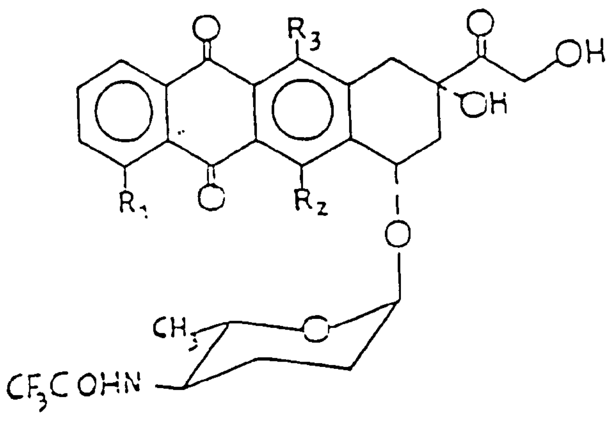

8

wherein $R_1$, $R_2$ and $R_3$ are as defined above ;

(iii') converting the compound of formula 8 into a 9,14-orthoformate derivative of formula 9 :

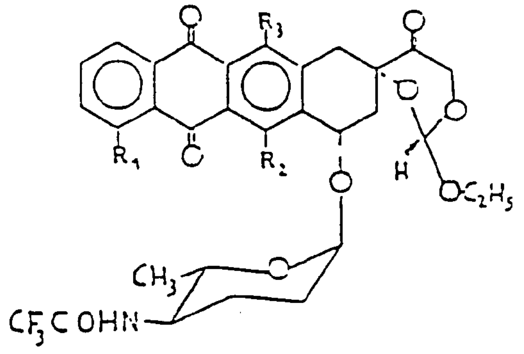

9

wherein $R_1$, $R_2$ and $R_3$ are as defined above :

(iv') removing the N-trifluoroacetyl group and the orthoformate protecting group to obtain a said glycoside of formula 2 ; and

(v') if desired, converting the said glycoside of formula 2 into a pharmaceutically acceptable acid addition salt thereof.

6. A process according to claim 5, wherein in step (i') the aglycone of formula 6 is reacted at room temperature with a 1-chloro-hexapyranoside of formula 4 in the presence of a molecular sieve and silver trifluoromethansulfonate.

7. A process according to claim 5 or 6, wherein in step (ii') the compound of formula 7 is treated at 0°C, for four hours and under a nitrogen atmosphere with potassium carbonate.

8. A process according to any one of claims 5 to 7, wherein in step (iii') the compound of formula 8 is treated with triethyl orthoformate.

9. A process according to claim 8, wherein the compound of formula 8 is treated with the triethyl orthoformate in methylene chloride and in the presence of pyridinium p-toluensulfonate for one hour at room temperature.

10. A process according to any one of claims 5 to 9, wherein in step (iv') the compound of formula 9 is subjected to mild alkaline aqueous hydrolysis to remove the N-trifluoroacetyl group and is treated with acetic acid to remove the orthoformate protecting group to obtain the said glycoside of formula 2 as a free base which, in step (v'), is treated with anhydrous methanolic hydrogen chloride to isolate the glycoside as its hydrochloride salt.

11. A process for the preparation of an anthracycline glycoside of formula 2 according to claim 1 wherein $R_1$ is as defined in claim 1 and one of $R_2$ and $R_3$ is hydroxy and the other of $R_2$ and $R_3$ is an amino group, or a pharmaceutically acceptable acid addition salt thereof, which process comprises :

(i'') reducing the C-6 or C-11 nitro group of a 9,14-orthoformate derivative of formula 9 as defined in claim 5 wherein one of $R_2$ and $R_3$ is hydroxy and the other of $R_2$ and $R_3$ is a nitro group ;

(ii'') removing the N-trifluoroacetyl group and the orthoformate protecting group from the C-6 or C-11 amino group-containing compound thus formed to obtain a said glycoside of formula 2 ; and

(iii") if desired, converting the said glycoside of formula 2 into a pharmaceutically acceptable acid addition salt thereof.

12. A process according to claim 11, wherein step (i") is effected by refluxing the 9,14-orthoformate derivative of formula 9 in a mixture of methanol and cyclohexene in the presence of 10% Pd/C for ten minutes.

13. A process according to claim 11 or 12, wherein the N-trifluoroacetyl group is removed in step (ii") in a basic medium and the orthoformate protecting group is removed with acetic acid to give a said glycoside of formula 2 which is converted in step (iii") by treatment with anhydrous methanolic hydrogen chloride into its hydrochloride.

## Patentansprüche

### Patentansprüche für folgende Vertragsstaaten : AT, BE, CH, DE, DK, FR, GB, IT, LI, NL, SE

1. Anthracyclinglycosid der allgemeinen Formel (1) oder (2)

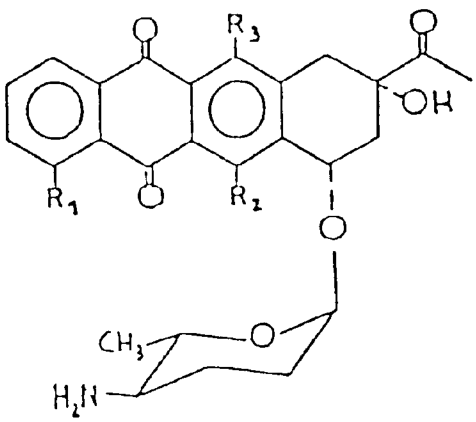

(1)

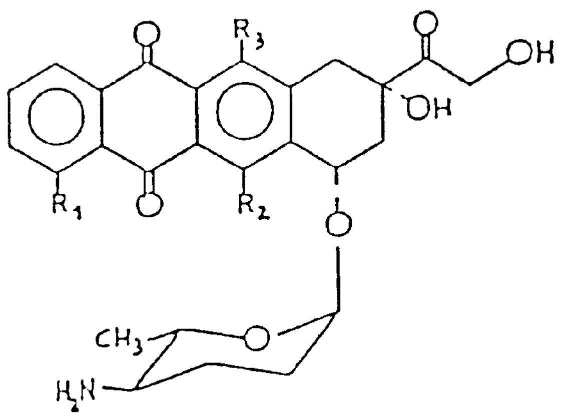

(2)

wobei $R_1$ aus der Reihe Wasserstoff, Fluor, Hydroxygruppe oder Aminogruppe ausgewählt ist ; $R_2$ und $R_3$ beide eine Hydroxygruppe darstellen, oder einer der Substituenten $R_2$ und $R_3$ Wasserstoff, eine Nitrogruppe oder eine Aminogruppe ist, und der andere der Substituenten $R_2$ und $R_3$ eine Hydroxygruppe ist, unter der Voraussetzung, dass, wenn $R_2$ und $R_3$ beide eine Hydroxygruppe sind, dann $R_1$ Fluor, eine Hydroxygruppe oder eine Aminogruppe darstellt ; und pharmazeutisch verträgliche Säureadditionssalze davon.

2. Verbindung nach Anspruch 1, die als Anthracyclinglycosid der Formel (1) 4-Demethyl-3′-deamino-4′-deoxy-4′-epi-amino-daunorubicin oder das Hydrochlorid ist.

3. Verbindung nach Anspruch 1, die als Anthracyclinglycosid der Formel (1) 4-Demethoxy-4-amino-3′-deamino-4′-deoxy-4′-epi-amino-daunorubicin oder das Hydrochlorid ist.

4. Verbindung nach Anspruch 1, die als Anthracyclinglycosid der Formel (1) 4-Demethoxy-4-fluoro-3′-deamino-4′-deoxy-4′-epi-amino-daunorubicin oder das Hydrochlorid ist.

5. Verbindung nach Anspruch 1, die als Anthracyclinglycosid der Formel (2) 4-Demethyl-3′-deamino-4′-deoxy-4′-epi-amino-doxorubicin oder das Hydrochlorid ist.

6. Verbindung nach Anspruch 1, die als Anthracyclinglycosid der Formel (2) 4-Demethyl-6-deoxy-3′-deamino-4′-deoxy-4′-epi-amino-doxorubicin oder das Hydrochlorid ist.

7. Verbindung nach Anspruch 1, die als Anthracyclinglycosid der Formel (2) 4-Demethoxy-11-deoxy-11-nitro-3′-deamino-4′-deoxy-4′-epi-amino-doxorubicin oder das Hydrochlorid ist.

8. Verbindung nach Anspruch 1, die als Anthracyclinglycosid der Formel (2) 4-Demethoxy-11-deoxy-11-amino-3′-deamino-4′-deoxy-4′-epi-amino-doxorubicin oder das Hydrochlorid ist.

9. Verbindung nach Anspruch 1, die als Anthracyclinglycosid der Formel (2) 4-Demethoxy-6-deoxy-6-nitro-3′-deamino-4′-deoxy-4′-epi-amino-doxorubicin oder das Hydrochlorid ist.

10. Verbindung nach Anspruch 1, die als Anthracyclinglycosid der Formel (2) 4-Demethoxy-6-deoxy-6-amino-3′-deamino-4′-deoxy-4′-epi-amino-doxorubicin oder das Hydrochlorid ist.

11. Verfahren zur Herstellung eines Anthracyclinglycosides der Formel (1) oder (2), wie in Anspruch 1 definiert, unter der Voraussetzung, dass für ein Glycosid der Formel (2) weder $R_2$ noch $R_3$ eine Aminogruppe darstellen, oder eines pharmazeutisch verträglichen Säureadditionssalzes davon, wobei das Verfahren die Schritte umfasst :

(i) Kondensieren eines Aglycons der Formel (3)

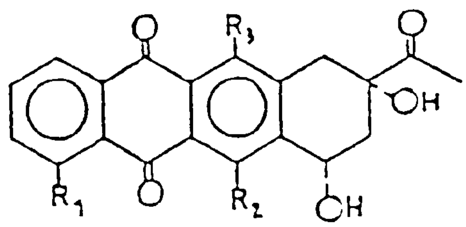

(3)

wobei $R_1$, $R_2$ und $R_3$ wie in Anspruch 1 definiert sind, mit der Ausnahme, dass weder $R_2$ noch $R_3$ eine Aminogruppe darstellen, mit einem 1-Halo-2,3,4,6-tetradeoxy-4-(N-trifluoroacetamido)-L-erythro-hexopyranosid der Formel (4)

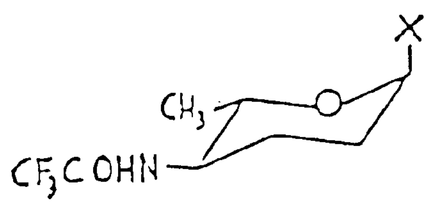

(4)

wobei X Halogen ist ;

(ii) Entfernen der N-Trifluoroacetylgruppe von der so erhaltenen Verbindung der Formel (5)

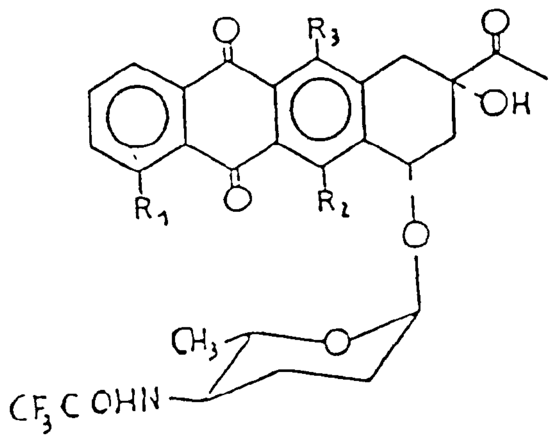

(5)

wobei $R_1$, $R_2$ und $R_3$ wie in Schritt (i) definiert sind, unter Erhalt eines Anthracyclinglycosides der Formel (1), mit der Ausnahme, dass weder $R_2$ noch $R_3$ eine Aminogruppe darstellen ;

(iii) gegebenenfalls Umwandeln des in Schritt (ii) erhaltenen Glycosides der Formel (1) in ein pharmazeutisch verträgliches Säureadditionssalz davon ;

(iv) gegebenenfalls Reduzieren des in Schritt (ii) erhaltenen Glycosides der Formel (1), wobei einer der Substituenten $R_2$ und $R_3$ eine Nitrogruppe ist, oder eines in Schritt (iii) erhaltenen Salzes davon, unter Erhalt eines Glycosides der Formel (1), wobei einer der Substituenten $R_2$ und $R_3$ eine Aminogruppe darstellt, und, gegebenenfalls Umwandeln des Glycosides der Formel (1), wobei einer der Substituenten $R_2$ und $R_3$ eine Aminogruppe ist, in ein pharmazeutisch verträgliches Säureadditionssalz davon ;

(v) gegebenenfalls Bromieren des in Schritt (ii) erhaltenen Glycosides der Formel (1) oder des in Schritt (iii) erhaltenen pharmazeutisch verträglichen Säureadditionssalzes davon, und Hydrolysieren des so erhaltenen 14-Bromoderivates, unter Bildung eines entsprechendes Anthracyclinglycosides der Formel (2), wie oben definiert ; und

(vi) gegebenenfalls Umwandeln des Glycosides der Formel (2) in ein pharmazeutisch verträgliches Säureadditionssalz davon.

12. Verfahren nach Anspruch 11, wobei in Schritt (i) das Aglycon der Formel (3) bei Raumtemperatur mit einem 1-Chloro-hexopyranosid der Formel (4) in Gegenwart eines Molekularsiebes und von Silbertrifluormethansulfonat umgesetzt wird.

13. Verfahren nach Anspruch 11 oder 12, wobei in Schritt (ii) die Verbindung der Formel (5), gelöst in Aceton, bei einer Temperatur von 0°C und während der Dauer von 1 Stunde einer milden alkalischen Hydrolyse mit 0,2 N wässrigem Natriumhydroxid unterzogen wird, um ein Glycosid der Formel (1) zu erhalten.

14. Verfahren gemäss einem der Ansprüche 11 bis 13, wobei das Glycosid der Formel (1), gelöst in einer Mischung von wasserfreiem Methanol und Dioxan, in Schritt (v) mit einer Chloroformlösung von Brom behandelt wird, um das entsprechende 14-Bromderivat zu erhalten, das 2 Tage lang bei Raumtemperatur mit einer wässrigen Lösung von Natriumformiat hydrolysiert wird, um ein Glycosid der Formel (2) als freie Base zu erhalten, und wobei in Schritt (vi) das Glycosid der Formel (2) als Hydrochlorid isoliert wird.

15. Verfahren zur Herstellung eines Anthracyclinglycosides der Formel (2), wie in Anspruch 1 definiert, unter der Voraussetzung, dass weder $R_2$ noch $R_3$ eine Aminogruppe darstellen, oder eines pharmazeutisch verträglichen Säureadditionssalzes davon, wobei das Verfahren die Schritte umfasst :

(i') Kondensieren eines 14-geschützten Aglycons der Formel (6)

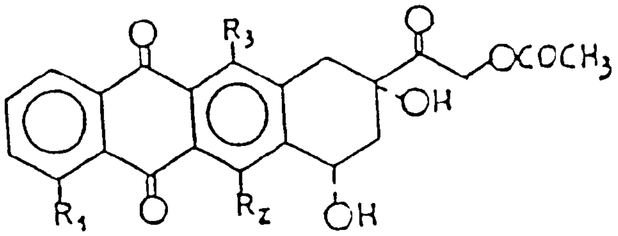

(6)

wobei $R_1$, $R_2$ und $R_3$ wie in Anspruch 1 definiert sind, mit der Ausnahme, dass weder $R_2$ noch $R_3$ eine Aminogruppe darstellen, mit einem 1-Halo-2,3,4,6-tetradeoxy-4-(N-trifluoroacetamido)-L-erythro-hexopyranosid der Formel (4), wie in Anspruch 11 definiert ;

(ii') Entfernen der Schutzgruppe in der Position 14 aus dem erhaltenen N-geschützten Glycosid der Formel (7)

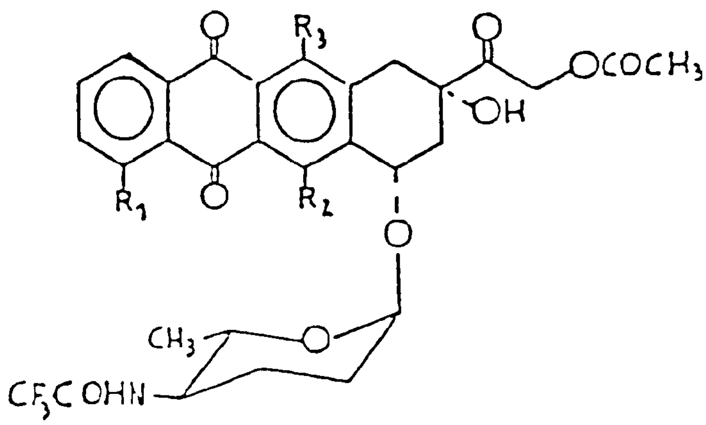

(7)

wobei $R_1$, $R_2$ und $R_3$ wie oben definiert sind, unter Erhalt der Verbindung der Formel (8)

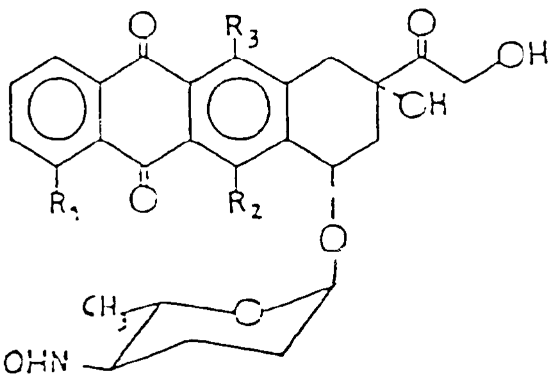

(8)

wobei $R_1$, $R_2$ und $R_3$ wie oben definiert sind ;

(iii′) Umwandeln der Verbindung der Formel (8) in ein 9,14-Orthoformiatderivat der Formel (9)

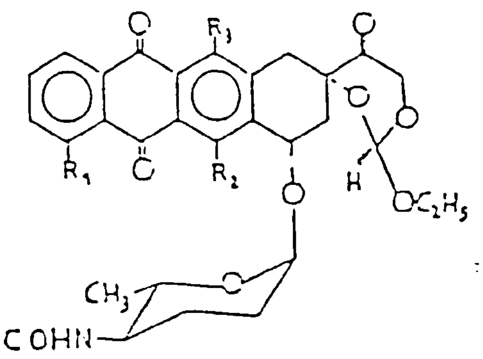

(9)

wobei $R_1$, $R_2$ und $R_3$ wie oben definiert sind ;

(iv′) Entfernen der N-Trifluoroacetylgruppe und der Orthoformiat-Schutzgruppe, unter Erhalt eines Glycosides der Formel (2) ; und

(v′) gegebenenfalls Umwandeln des Glycosides der Formel (2) in ein pharmazeutisch verträgliches Säureadditionssalz davon.

16. Verfahren gemäss Anspruch 15, wobei in Schritt (i′) das Aglycon der Formel (6) bei Raumtemperatur mit einem 1-Chloro-hexapyranosid der Formel (4) in Gegenwart eines Molekularsiebes und von Silbertrifluoromethansulfonat umgesetzt wird.

17. Verfahren nach Anspruch 15 oder 16, wobei in Schritt (ii′) die Verbindung der Formel (7) bei 0°C für 4 Stunden und unter einer Stickstoffatmosphäre mit Kaliumkarbonat behandelt wird.

18. Verfahren gemäss einem der Ansprüche 15 bis 17, wobei in Schritt (iii′) die Verbindung der Formel (8) mit Triethylorthoformiat behandelt wird.

19. Verfahren gemäss Anspruch 18, wobei die Verbindung der Formel (8) mit Triethylorthoformiat in Methylenchlorid und in Gegenwart von Pyridinium-p-toluolsulfonat 1 Stunde lang bei Raumtemperatur behandelt wird.

20. Verfahren gemäss einem der Ansprüche 15 bis 19, wobei in Schritt (iv′) die Verbindung der Formel (9) einer milden alkalischen, wässrigen Hydrolyse unterzogen wird, um die N-Trifluoroacetylgruppe zu entfernen, und sie mit Essigsäure behandelt wird, um die Orthoformiat-Schutzgruppe zu entfernen, wobei das Glycosid der Formel (2) als freie Base erhalten wird, das in Schritt (v′) mit wasserfreiem, methanolischen Wasserstoffchlorid behandelt wird, um das Glycosid als Hydrochloridsalz zu isolieren.

21. Verfahren zur Herstellung eines Anthracyclinglycosides der Formel (2) gemäss Anspruch 1, wobei $R_1$ wie in Anspruch 1 definiert ist und einer der Substituenten $R_2$ und $R_3$ eine Hydroxygruppe ist und der andere Substituent von $R_2$ und $R_3$ eine Aminogruppe ist, oder eines pharmazeutisch verträglichen Säureadditionssalzes davon, wobei das Verfahren die Schritte umfasst :

(i″) Reduzieren der C-6- oder C-11-Nitrogruppe eines 9,14-Orthoformiatderivates der Formel (9), wie in Anspruch 15 definiert, wobei einer der Substituenten von $R_2$ und $R_3$ eine Hydroxygruppe ist, und der andere Substituent von $R_2$ und $R_3$ eine Nitrogruppe ist ;

(ii″) Entfernen der N-Trifluoroacetylgruppe und der Orthoformiat-Schutzgruppe von der C-6- oder C-11-aminogruppenhaltigen Verbindung, die so gebildet wurde, um ein Glycosid der Formel (2) zu erhalten ; und

(iii") gegebenenfalls Umwandeln des Glycosides der Formel (2) in ein pharmazeutisch verträgliches Säureadditionssalz davon.

22. Verfahren nach Anspruch 21, wobei der Schritt (i") durchgeführt wird, indem man das 9,14-Orthoformiatderivat der Formel (9) in einer Mischung von Methanol und Cyclohexen in Gegenwart von 10% Pd/C 10 Minuten lang refluxiert.

23. Verfahren gemäss Anspruch 21 oder 22, wobei die N-Trifluoroacetylgruppe in Schritt (i") in einem basischen Medium entfernt wird, und wobei die Orthoformiat-Schutzgruppe mit Essigsäure entfernt wird, wobei man ein Glycosid der Formel (2) erhält, das in Schritt (ii") durch Behandeln mit wasserfreiem, methanolischen Wasserstoffchlorid in das Hydrochlorid umgewandelt wird.

24. Pharmazeutische Zusammensetzung, umfassend ein Anthracyclinglycosid der Formeln (1) und (2), wie in Anspruch 1 definiert, oder eines pharmazeutisch verträglichen Säureadditionssalzes davon, zusammen mit einem pharmazeutisch verträglichen Träger oder Streckmittel.

25. Anthracyclinglycosid der Formeln (1) und (2), wie in Anspruch 1 definiert, oder eines pharmazeutisch verträglichen Säureadditionssalzes davon, zur Anwendung in einem Verfahren zur Behandlung des menschlichen oder tierischen Körpers mittels Therapie.

26. Verbindung nach Anspruch 25 zur Verwendung als Anti-Tumormittel.

27. Verbindung der Formel (5), wie in Anspruch 11 definiert.

28. Verbindung der Formeln (7), (8) oder (9), wie in Anspruch 15 definiert.

**Patentansprüche für folgende Vertragsstaaten : ES, GR**

1. Verfahren zur Herstellung eines Anthracyclinglycosides der Formel (1) oder (2)

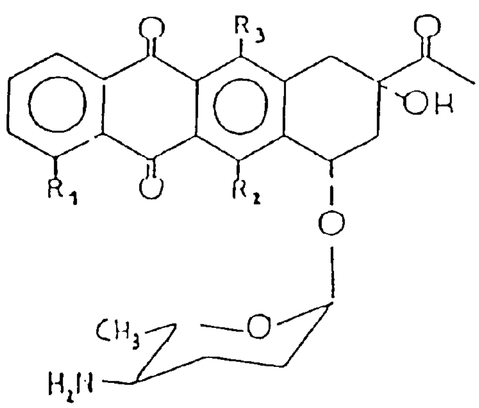

(1)

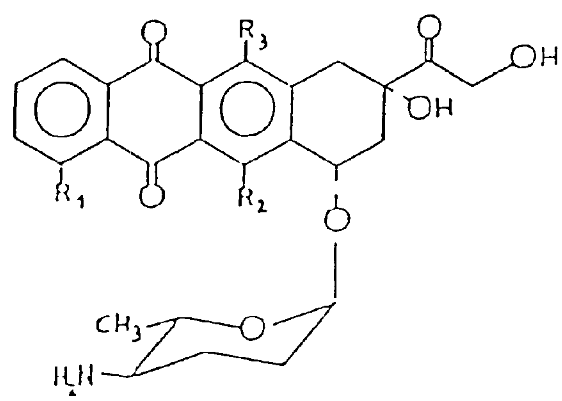

(2)

wobei $R_1$ aus der Reihe Wasserstoff, Fluor, Hydroxy- und Aminogruppe ausgewählt ist ; $R_2$ und $R_3$ beide eine Hydroxygruppe darstellen oder einer der Substituenten $R_2$ und $R_3$ Wasserstoff, eine Nitro- oder Aminogruppe ist, und der andere der Substituenten $R_2$ und $R_3$ eine Hydroxygruppe darstellt ; unter der Voraussetzung, dass, wenn $R_2$ und $R_3$ beide eine Hydroxygruppe sind, dann $R_1$ Fluor, eine Hydroxy- oder Aminogruppe ist, und von pharmazeutisch verträglichen Säureadditionssalzen davon, wobei das Verfahren die Schritte umfasst :

(i) Kondensieren eines Aglycons der Formel (3)

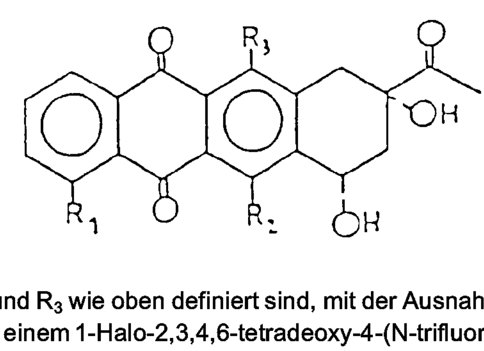

(3)

wobei $R_1$, $R_2$ und $R_3$ wie oben definiert sind, mit der Ausnahme, dass weder $R_2$ noch $R_3$ eine Aminogruppe darstellen, mit einem 1-Halo-2,3,4,6-tetradeoxy-4-(N-trifluoroacetamido)-L-erythro-hexopyranosid der For-

mel (4)

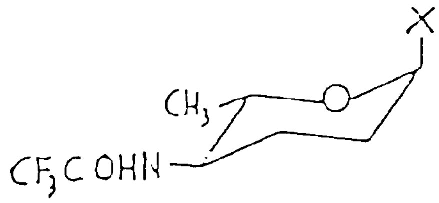

(4)

wobei X Halogen ist ;
(ii) Entfernen der N-Trifluoroacetylgruppe von der so erhaltenen Verbindung der Formel (5)

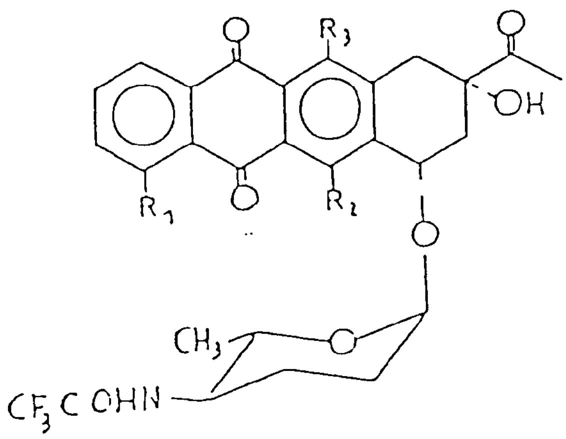

(5)

wobei $R_1$, $R_2$ und $R_3$ wie in Schritt (i) definiert sind, unter Erhalt eines Anthracyclinglycosides der Formel (1), mit der Ausnahme, dass weder $R_2$ noch $R_3$ eine Aminogruppe darstellen ;
(iii) gegebenenfalls Umwandeln des in Schritt (ii) erhaltenen Glycosides der Formel (1) in ein pharmazeutisch verträgliches Säureadditionssalz davon ;
(iv) gegebenenfalls Reduzieren des in Schritt (ii) erhaltenen Glycosides der Formel (1), wobei einer der Substituenten $R_2$ und $R_3$ eine Nitrogruppe ist, oder eines in Schritt (iii) erhaltenen Salzes davon, unter Erhalt eines Glycosides der Formel (1), wobei einer der Substituenten $R_2$ und $R_3$ eine Aminogruppe darstellt, und, gegebenenfalls Umwandeln des Glycosides der Formel (1), wobei einer der Substituenten $R_2$ und $R_3$ eine Aminogruppe ist, in ein pharmazeutisch verträgliches Säureadditionssalz davon ;
(v) gegebenenfalls Bromieren des in Schritt (ii) erhaltenen Glycosides der Formel (1) oder des in Schritt (iii) erhaltenen pharmazeutisch verträglichen Säureadditionssalzes davon, und Hydrolysieren des so erhaltenen 14-Bromderivates, unter Bildung eines entsprechendes Anthracyclinglycosides der Formel (2), wie oben definiert ; und
(vi) gegebenenfalls Umwandeln des Glycosides der Formel (2) in ein pharmazeutisch verträgliches Säureadditionssalz davon.

2. Verfahren nach Anspruch 1, wobei in Schritt (i) das Aglycon der Formel (3) bei Raumtemperatur mit einem 1-Chloro-hexopyranosid der Formel (4) in Gegenwart eines Molekularsiebes und von Silbertrifluormethansulfonat umgesetzt wird.

3. Verfahren nach Anspruch 1 oder 2, wobei in Schritt (ii) die Verbindung der Formel (5), gelöst in Aceton, bei einer Temperatur von 0°C und während 1 Stunde einer milden alkalischen Hydrolyse mit 0,2 N wässrigem Natriumhydroxid unterzogen wird, um ein Glycosid der Formel (1) zu erhalten.

4. Verfahren gemäss einem der Ansprüche 1 bis 3, wobei das Glycosid der Formel (1), gelöst in einer Mischung von wasserfreiem Methanol und Dioxan, in Schritt (v) mit einer Chloroformlösung von Brom behandelt wird, um das entsprechende 14-Bromderivat zu erhalten, das 2 Tage lang bei Raumtemperatur mit einer wässrigen Lösung von Natriumformiat hydrolysiert wird, um ein Glycosid der Formel (2) als freie Base zu erhalten, und wobei in Schritt (vi) das Glycosid der Formel (2) als Hydrochlorid isoliert wird.

5. Verfahren zur Herstellung eines Anthracyclinglycosides der Formel (2), gemäss der Definition in Anspruch 1, unter der Voraussetzung, dass weder $R_2$ noch $R_3$ eine Aminogruppe darstellen, oder eines pharmazeutisch verträglichen Säureadditionssalzes davon, wobei das Verfahren die Schritte umfasst :
(i') Kondensieren eines 14-geschützten Aglycons der Formel (6)

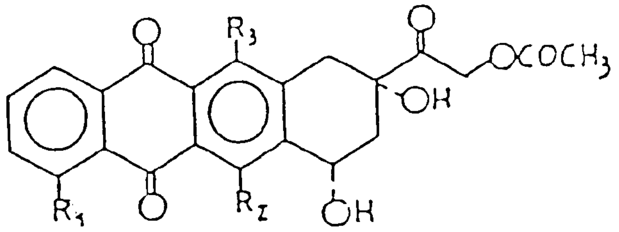

(6)

wobei $R_1$, $R_2$ und $R_3$ wie in Anspruch 1 definiert sind, mit der Ausnahme, dass weder $R_2$ noch $R_3$ eine Aminogruppe darstellen, mit einem 1-Halo-2,3,4,6-tetradeoxy-4-(N-trifluoroacetamido)-L-erythro-hexopyranosid der Formel (4), wie in Anspruch 1 definiert ;

(ii') Entfernen der 14-Schutzgruppe von dem erhaltenen N-geschützten Glycosid der Formel (7)

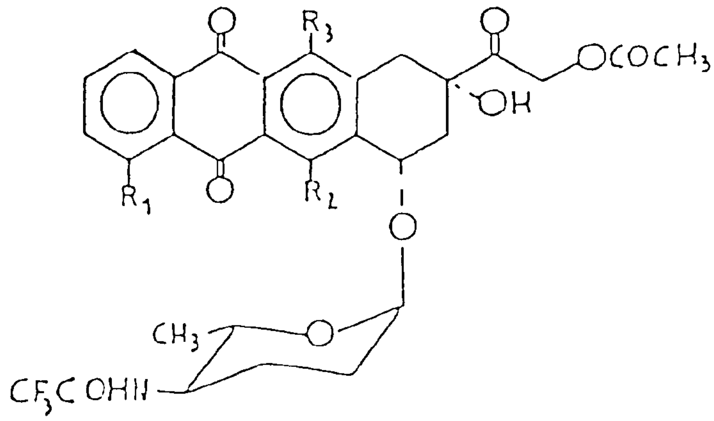

(7)

wobei $R_1$, $R_2$ und $R_3$ wie oben definiert sind, unter Erhalt der Verbindung der Formel (8)

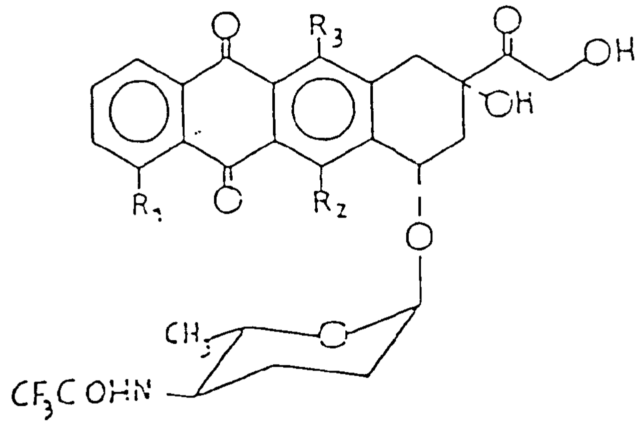

(8)

wobei $R_1$, $R_2$ und $R_3$ wie oben definiert sind ;

(iii') Umwandeln der Verbindung der Formel (8) in ein 9,14-Orthoformiatderivat der Formel (9)

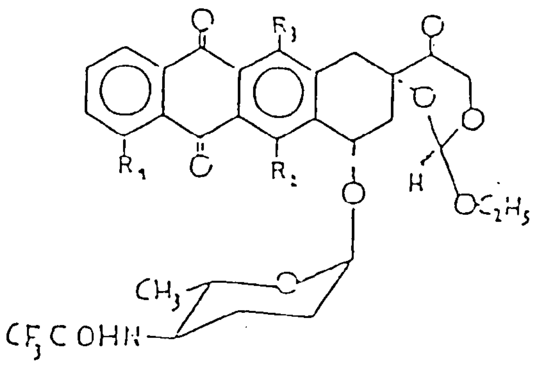

(9)

wobei $R_1$, $R_2$ und $R_3$ wie oben definiert sind ;

(iv') Entfernen der N-Trifluoroacetylgruppe und der Orthoformiat-Schutzgruppe, unter Erhalt eines Glycosides der Formel (2) ; und

(v') gegebenenfalls Umwandeln des Glycosides der Formel (2) in ein pharmazeutisch verträgliches Säureadditionssalz davon.

6. Verfahren gemäss Anspruch 5, wobei in Schritt (i') das Aglycon der Formel (6) bei Raumtemperatur mit einem 1-Chloro-hexapyranosid der Formel (4) in Gegenwart eines Molekularsiebes und von Silbertrifluoromethansulfonat umgesetzt wird.

7. Verfahren nach Anspruch 5 oder 6, wobei in Schritt (ii') die Verbindung der Formel (7) bei 0°C 4 Stunden lang unter einer Stickstoffatmosphäre mit Kaliumkarbonat behandelt wird.

8. Verfahren gemäss einem der Ansprüche 5 bis 7, wobei in Schritt (iii') die Verbindung der Formel (8) mit Triethylorthoformiat behandelt wird.

9. Verfahren gemäss Anspruch 8, wobei die Verbindung der Formel (8) mit dem Triethylorthoformiat in Methylenchlorid und in Gegenwart von Pyridinium-p-toluolsulfonat 1 Stunde lang bei Raumtemperatur behandelt wird.

10. Verfahren gemäss einem der Ansprüche 5 bis 9, wobei in Schritt (iv') die Verbindung der Formel (9) einer milden alkalischen, wässrigen Hydrolyse unterzogen wird, um die N-Trifluoroacetylgruppe zu entfernen, und mit Essigsäure behandelt wird, um die Orthoformiat-Schutzgruppe zu entfernen, wobei das Glycosid der Formel (2) als freie Base erhalten wird, das in Schritt (v') mit wasserfreiem, methanolischen Wasserstoffchlorid behandelt wird, um das Glycosid als Hydrochloridsalz zu isolieren.

11. Verfahren zur Herstellung eines Anthracyclinglycosides der Formel (2) gemäss Anspruch 1, wobei $R_1$ wie in Anspruch 1 definiert ist und einer der Substituenten $R_2$ und $R_3$ eine Hydroxygruppe ist und der andere der Substituenten $R_2$ und $R_3$ eine Aminogruppe darstellt, oder eines pharmazeutisch verträglichen Säureadditionssalzes davon, wobei das Verfahren die Schritte umfasst :

(i") Reduzieren der C-6- oder C-11-Nitrogruppe eines 9,14-Orthoformiatderivates der Formel (9), wie in Anspruch 5 definiert, wobei einer der Substituenten $R_2$ und $R_3$ eine Hydroxygruppe und der andere eine Nitrogruppe ist ;

(ii") Entfernen der N-Trifluoroacetylgruppe und der Orthoformiat-Schutzgruppe von der C-6- oder C-11-aminogruppenhaltigen Verbindung, die so gebildet wurde, um ein Glycosid der Formel (2) zu erhalten ; und

(iii") gegebenenfalls Umwandeln des Glycosides der Formel (2) in ein pharmazeutisch verträgliches Säureadditionssalz davon.

12. Verfahren nach Anspruch 11, wobei der Schritt (i") durchgeführt wird, indem man das 9,14-Orthoformiatderivat der Formel (9) in einer Mischung von Methanol und Cyclohexen in Gegenwart von 10% Pd/C 10 Minuten lang refluxiert.

13. Verfahren gemäss Anspruch 11 oder 12, wobei die N-Trifluoroacetylgruppe in Schritt (i") in einem basischen Medium entfernt wird, und die Orthoformiat-Schutzgruppe mit Essigsäure entfernt wird, um man ein Glycosid der Formel (2) zu erhalten, das in Schritt (ii") durch Behandeln mit wasserfreiem, methanolischen Wasserstoffchlorid in das Hydrochlorid umgewandelt wird.

## Revendications

### Revendications pour les Etats contractants suivants : AT, BE, CH, DE, DK, FR, GB, IT, LI, NL, SE

1. Anthracycline glycoside de formule générale 1 ou 2 :

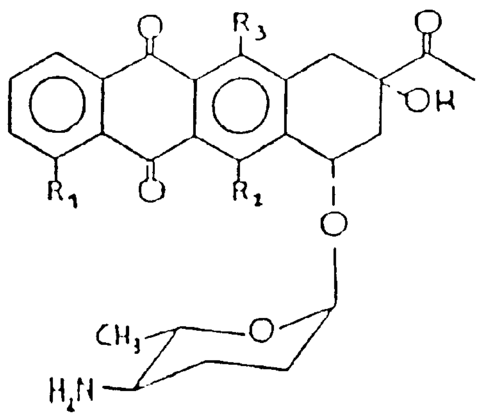

1

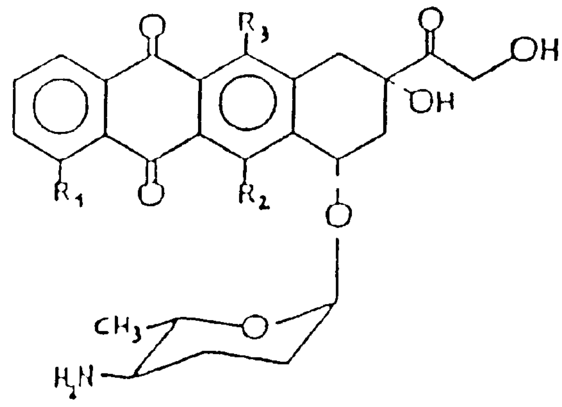

2

dans lesquelles
— $R_1$ est choisi dans le groupe constitué par hydrogène, fluor, hydroxy ou amino ;
— $R_2$ et $R_3$ représentent tous deux hydroxy, ou bien l'un parmi $R_2$ et $R_3$ représente hydrogène, nitro ou amino, et l'autre parmi $R_2$ et $R_3$ représente hydroxy ; à la condition que, lorsque $R_2$ et $R_3$ représentent tous deux hydroxy, $R_1$ représente fluor, hydroxy ou amino ;
et ses sels pharmaceutiquement acceptables d'addition avec un acide.

2. Composé selon la revendication 1, qui est l'anthracycline glycoside de formule 1 : déméthyl-4 désamino-3′ désoxy-4′ épi-amino-4′ daunorubicine, ou son chlorhydrate.

3. Composé selon la revendication 1, qui est l'anthracycline glycoside de formule 1 : déméthoxy-4 amino-4 désamino-3′ désoxy-4′ épi-amino-4′ daunorubicine, ou son chlorhydrate.

4. Composé selon la revendication 1, qui est l'anthracycline glycoside de formule 1 : déméthoxy-4 fluoro-4 désamino-3′ désoxy-4′ épi-amino-4′ daunorubicine, ou son chlorhydrate.

5. Composé selon la revendication 1, qui est l'anthracycline glycoside de formule 2 : déméthyl-4 désamino-3′ désoxy-4′ épi-amino-4′ doxorubicine, ou son chlorhydrate.

6. Composé selon la revendication 1, qui est l'anthracycline glycoside de formule 2 : déméthyl-4 désoxy-6 désamino-3′ désoxy-4′ épi-amino-4′ doxorubicine, ou son chlorhydrate.

7. Composé selon la revendication 1, qui est l'anthracycline glycoside de formule 2 : déméthoxy-4 désoxy-11 nitro-11 désamino-3′ désoxy-4′ épi-amino-4′ doxorubicine, ou son chlorhydrate.

8. Composé selon la revendication 1, qui est l'anthracycline glycoside de formule 2 : déméthoxy-4 désoxy-11 amino-11 désamino-3′ désoxy-4′ épi-amino-4′ doxorubicine, ou son chlorhydrate.

9. Composé selon la revendication 1, qui est l'anthracycline glycoside de formule 2 : déméthoxy-4 désoxy-6 nitro-6 désamino-3′ désoxy-4′ épi-amino-4′ doxorubicine, ou son chlorhydrate.

10. Composé selon la revendication 1, qui est l'anthracycline glycoside de formule 2 : déméthoxy-4 désoxy-6 amino-6 désamino-3′ désoxy-4′ épi-amino-4′ doxorubicine, ou son chlorhydrate.

11. Procédé de préparation d'un anthracycline glycoside de la formule 1 ou 2 telle que définie à la revendication 1, à la condition que, pour un glycoside de formule 2, ni $R_2$ ni $R_3$ ne représentent un groupe amino ou d'un de ses sels pharmaceutiquement acceptables d'addition avec un acide, ledit procédé comprenant :

(i) la condensation d'une aglycone de formule 3

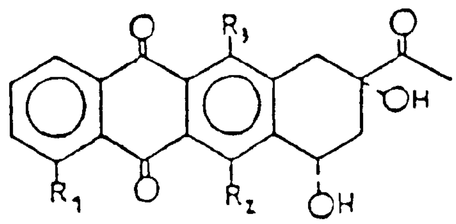

3

dans laquelle $R_1$, $R_2$ et $R_3$ sont tels que définis à la revendication 1, excepté que ni $R_2$ ni $R_3$ ne représentent un groupe amino, avec un halo-1 tétradésoxy-2,3,4,6 (N-trifluoroacétamido)-4 L-érythro-hexopyranoside de formule 4 :

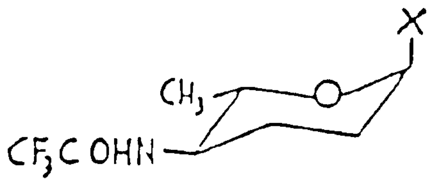

4

dans laquelle X représente halogène ;
(ii) l'élimination du groupe N-trifluoroacétyle du composé ainsi obtenu de formule 5 :

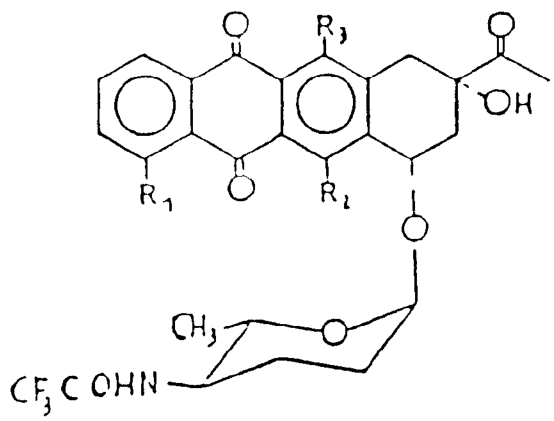

5

dans laquelle $R_1$, $R_2$ et $R_3$ sont tels que définis à l'étape (i), de façon à obtenir un anthracycline glycoside précité de formule 1, excepté que ni $R_2$ ni $R_3$ ne représentent un groupe amino ;
(iii) si on le désire, la conversion dudit glycoside de formule 1, obtenu à l'étape (ii), en l'un de ses sels pharmaceutiquement acceptables d'addition avec un acide ;
(iv) si on le désire, la réduction d'un glycoside précité de formule 1, dans laquelle l'un parmi $R_2$ et $R_3$ représente un groupe nitro, obtenu à l'étape (ii) ou l'un de ses sels obtenu à l'étape (iii), de façon à obtenir un glycoside précité de la formule 1, dans laquelle l'un parmi $R_2$ et $R_3$ représente un groupe amino et, si on le désire, la conversion dudit glycoside de formule 1, dans laquelle l'un parmi $R_2$ et $R_3$ représente un groupe amino, en l'un de ses sels pharmaceutiquement acceptables d'addition avec un acide ;
(v) si on le désire, la bromation dudit glycoside de formule 1, obtenu à l'étape (ii), ou de l'un de ses sels pharmaceutiquement acceptables d'addition avec un acide, obtenu à l'étape (iii), et l'hydrolyse du dérivé bromo en position 14 ainsi obtenu, pour former un anthracycline glycoside correspondant de la formule 2 telle que définie ci-dessus ; et
(vi) si on le désire, la conversion dudit glycoside de formule 2 en l'un de ses sels pharmaceutiquement acceptables d'addition avec un acide.

12. Procédé selon la revendication 11, dans lequel, à l'étape (i), on fait réagir l'aglycone de formule 3 à la température ambiante avec un chloro-1 hexopyranoside de formule 4 en présence d'un tamis moléculaire et

de trifluorométhanesulfonate d'argent.

13. Procédé selon la revendication 11 ou 12, dans lequel, à l'étape (ii), on soumet, à une température de 0°C et pendant une heure, le composé de formule 5, dissous dans de l'acétone, à une hydrolyse alcaline modérée par de l'hydroxyde de sodium aqueux 0,2 N, pour donner un glycoside précité de formule 1.

14. Procédé selon l'une des revendications 11 à 13, dans lequel on traite, à l'étape (v), le glycoside précité de formule 1, dissous dans un mélange de méthanol et de dioxane anhydres, par une solution de brome dans le chloroforme, pour fournir le dérivé bromo en position 14 correspondant, lequel est hydrolysé pendant deux jours à la température ambiante par une solution aqueuse de formiate de sodium, pour donner un glycoside précité de formule 2 sous la forme d'une base libre et, à l'étape (vi), on isole ledit glycoside de formule 2 sous la forme de son chlorhydrate.

15. Procédé de préparation d'un anthracycline glycoside de la formule 2 telle que définie à la revendication 1, à la condition que ni $R_2$ ni $R_3$ ne représentent un groupe amino, ou d'un de ses sels pharmaceutiquement acceptables d'addition avec un acide, ledit procédé comprenant :

(i') la condensation d'une aglycone protégée en position 14 de formule 6 :

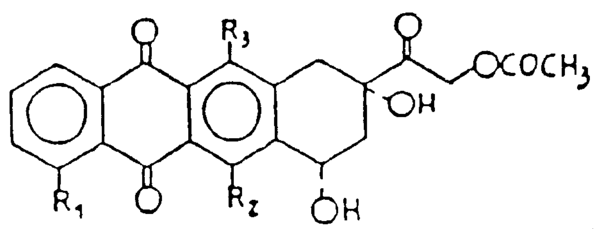

6

dans laquelle $R_1$, $R_2$ et $R_3$ sont tels que définis à la revendication 1, excepté que ni $R_2$ ni $R_3$ ne représentent un groupe amino,

avec un halo-1 tétradésoxy-2,3,4,6 (N-trifluoro-acétamido)-4 L-érythro-hexopyranoside de la formule 4 telle que définie à la revendication 11 ;

(ii') l'élimination du groupe protecteur en position 14 du glycoside N-protégé résultant de formule 7 :

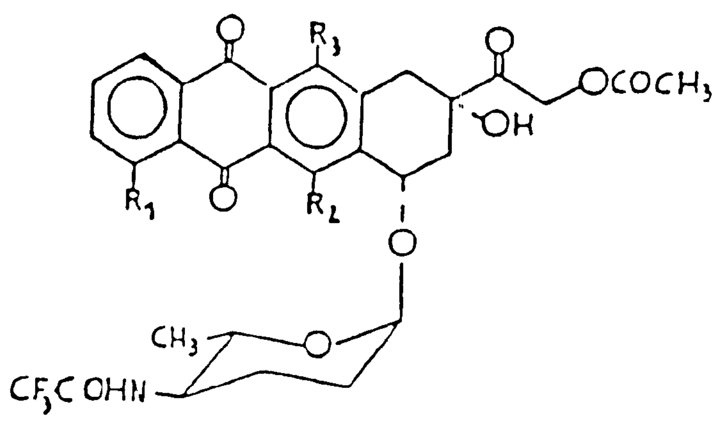

7

dans laquelle $R_1$, $R_2$ et $R_3$ sont tels que définis ci-dessus, pour donner le composé de formule 8 :

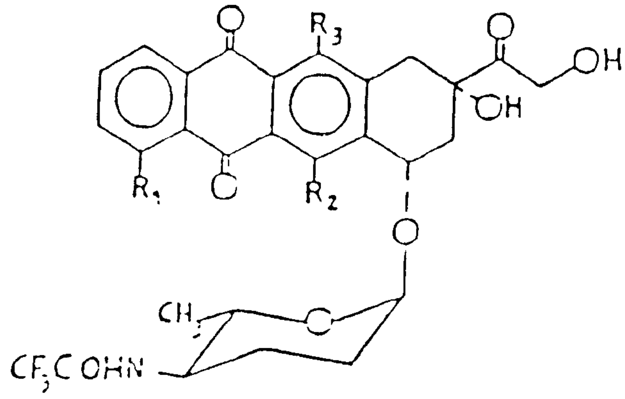

8

dans laquelle $R_1$, $R_2$ et $R_3$ sont tels que définis ci-dessus ;

(iii′) la conversion du composé de formule 8 en un dérivé d'orthoformiate-9,14 de formule 9 :

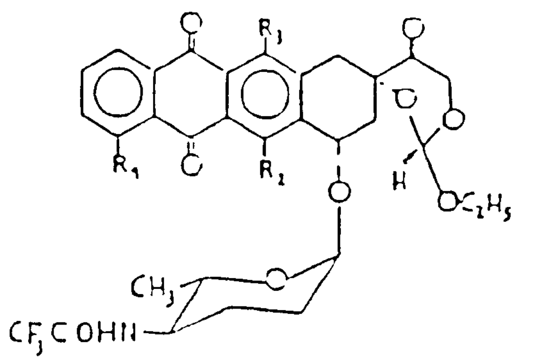

9

dans laquelle $R_1$, $R_2$ et $R_3$ sont tels que définis ci-dessus ;

(iv′) l'élimination du groupe N-trifluoroacétyle et du groupe protecteur orthoformiate, pour obtenir un glycoside précité de formule 2 ; et

(v′) si on le désire, la conversion dudit glycoside de formule 2 en l'un de ses sels pharmaceutiquement acceptables d'addition avec un acide.

16. Procédé selon la revendication 15, dans lequel, à l'étape (i′), on fait réagir, à la température ambiante, l'aglycone de formule 6 avec un chloro-1 hexapyranoside de formule 4, en présence d'un tamis moléculaire et de trifluorométhanesulfonate d'argent.

17. Procédé selon la revendication 15 ou 16, dans lequel, à l'étape (ii′), on traite le composé de formule 7, à 0°C, pendant quatre heures et sous atmosphère d'azote, par du carbonate de potassium.

18. Procédé selon l'une des revendications 15 à 17, dans lequel, à l'étape (iii′), on traite le composé de formule 8 par de l'orthoformiate de triéthyle.

19. Procédé selon la revendication 18, dans lequel on traite le composé de formule 8 par de l'orthoformiate de triéthyle dans du chlorure de méthylène et en présence de p-toluène sulfonate de pyridinium pendant une heure à la température ambiante.

20. Procédé selon l'une des revendications 15 à 19, dans lequel, à l'étape (iv′), on soumet le composé de formule 9 à une hydrolyse aqueuse alcaline modérée, pour éliminer le groupe N-trifluoroacétyle et on le traite par de l'acide acétique pour éliminer le groupe protecteur orthoformiate, afin d'obtenir ledit glycoside de formule 2 sous la forme d'une base libre, laquelle, à l'étape (v′), est traitée par du chlorure d'hydrogène méthanolique anhydre, pour isoler le glycoside sous la forme de son sel chlorhydrate.

21. Procédé de préparation d'un anthracycline glycosine de la formule 2 selon la revendication 1, dans laquelle $R_1$ est tel que défini dans la revendication 1, et l'un parmi $R_2$ et $R_3$ représente hydroxy et l'autre parmi $R_2$ et $R_3$ représente un groupe amino, ou d'un de ses sels pharmaceutiquement acceptables d'addition avec un acide, ledit procédé comprenant :

(i″) la réduction du groupe nitro en C-6 ou C-11 d'un dérivé d'orthoformiate-9,14 de la formule 9 telle que définie à la revendication 15, dans laquelle l'un parmi $R_2$ et $R_3$ représente hydroxy et l'autre parmi $R_2$ et $R_3$ représente un groupe nitro ;

(ii″) l'élimination du groupe N-trifluoroacétyle et du groupe protecteur orthoformiate à partir du composé

contenant un groupe amino en C-6 ou C-11 ainsi formé, pour obtenir un glycoside précité de formule 2 ; et

(iii") si on le désire, la conversion dudit glycoside de formule 2 en l'un de ses sels pharmaceutiquement acceptables d'addition avec un acide.

22. Procédé selon la revendication 21, dans lequel on effectue l'étape (i") en portant au reflux le dérivé d'orthoformiate-9,14 de formule 9 dans un mélange de méthanol et de cyclohexène, en présence de Pd/C à 10% pendant dix minutes.

23. Procédé selon la revendication 21 ou 22, dans lequel on élimine le groupe N-trifluoroacétyle à l'étape (ii") dans un milieu basique, et on élimine le groupe protecteur orthoformiate par de l'acide acétique, pour donner un diglycoside précité de formule 2, lequel est converti à l'étape (iii") en son chlorhydrate, par traitement par du chlorure d'hydrogène méthanolique anhydre.

24. Composition pharmaceutique comprenant un anthracycline glycoside de formule 1 et 2 telle que définie à la revendication 1, ou un sel pharmaceutiquement acceptable d'addition avec un acide, de ce composé, conjointement avec un support ou diluant pharmaceutiquement acceptable.

25. Anthracycline glycoside de formule 1 et 2, telle que définie à la revendication 1, ou sel pharmaceutiquement acceptable d'addition avec un acide, de ce composé, destiné à être utilisé dans une méthode de traitement du corps humain ou animal par thérapie.

26. Composé selon la revendication 25, destiné à être utilisé comme agent antitumoral.

27. Composé de formule 5 telle que définie à la revendication 11.

28. Composé de formule 7, 8 ou 9 telles que définies à la revendication 15.

**Revendications pour les Etats contractants suivants : ES, GR**

1. Procédé de préparation d'un anthracycline glycoside de la formule 1 ou 2 :

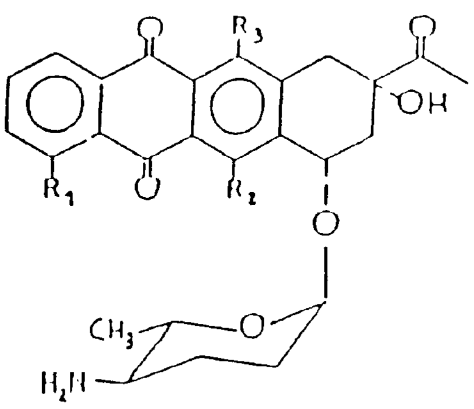

1

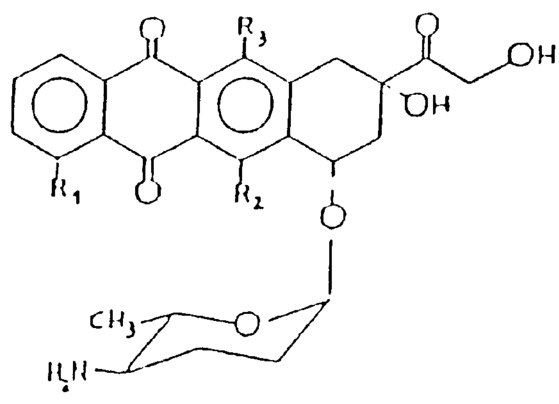

2

dans lesquelles

— $R_1$ est choisi dans le groupe constitué par hydrogène, fluor, hydroxy et amino ;

— $R_2$ et $R_3$ représentent tous deux hydroxy, ou bien l'un parmi $R_2$ et $R_3$ représente hydrogène, nitro ou amino, et l'autre parmi $R_2$ et $R_3$ représente hydroxy ; à la condition que, lorsque $R_2$ et $R_3$ représentent tous deux hydroxy, $R_1$ représente fluor, hydroxy ou amino ;

et de ses sels pharmaceutiquement acceptables d'addition avec un acide, ledit procédé comprenant :

(i) la condensation d'une aglycone de formule 3

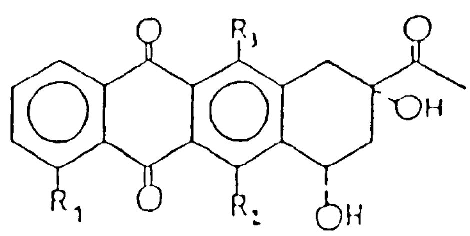

3

dans laquelle $R_1$, $R_2$ et $R_3$ sont tels que définis ci-dessus, excepté que ni $R_2$ ni $R_3$ ne représentent un groupe amino, avec un halo-1 tétradésoxy-2,3,4,6 (N-trifluoro-acétamido)-4 L-érythro-hexopyranoside de formule 4 :

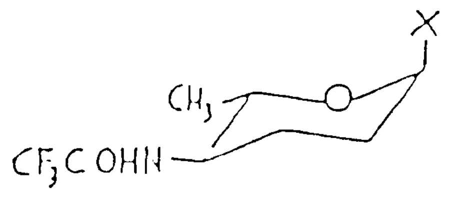

4

dans laquelle X représente halogène ;

(ii) l'élimination du groupe N-trifluoroacétyle du composé ainsi obtenu de formule 5 :

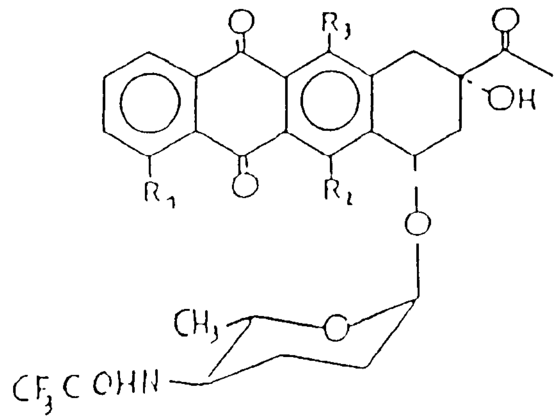

5

dans laquelle $R_1$, $R_2$ et $R_3$ sont tels que définis à l'étape (i), de façon à obtenir un anthracycline glycoside précité de formule 1, excepté que ni $R_2$ ni $R_3$ ne représentent un groupe amino ;

(iii) si on le désire, la conversion dudit glycoside de formule 1, obtenu à l'étape (ii), en l'un de ses sels pharmaceutiquement acceptables d'addition avec un acide ;

(iv) si on le désire, la réduction d'un glycoside précité de formule 1, dans laquelle l'un parmi $R_2$ et $R_3$ représente un groupe nitro, obtenu à l'étape (ii) ou d'un de ses sels, obtenu à l'étape (iii), de façon à obtenir un glycoside précité de formule 1, dans laquelle l'un parmi $R_2$ et $R_3$ représente un groupe amino et, si on le désire, la conversion dudit glycoside de formule 1, dans laquelle l'un parmi $R_2$ et $R_3$ représente un groupe amino, en l'un de ses sels pharmaceutiquement acceptables d'addition avec un acide ;

(v) si on le désire, la bromation dudit glycoside de formule 1, obtenu à l'étape (ii), ou de l'un de ses sels pharmaceutiquement acceptables d'addition avec un acide, obtenu à l'étape (iii), et l'hydrolyse du dérivé bromo en position 14 ainsi obtenu, pour former un anthracycline glycoside correspondant de formule 2 telle que définie ci-dessus ; et

(vi) si on le désire, la conversion dudit glycoside de formule 2 en l'un de ses sels pharmaceutiquement acceptables d'addition avec un acide.

2. Procédé selon la revendication 1, dans lequel, à l'étape (i), on fait réagir l'aglycone de formule 3 à la température ambiante avec un chloro-1 hexopyranoside de formule 4 en présence d'un tamis moléculaire et de trifluorométhanesulfonate d'argent.

3. Procédé selon la revendication 1 ou 2, dans lequel, à l'étape (ii), on soumet, à une température de 0°C et pendant une heure, le composé de formule 5, dissous dans de l'acétone, à une hydrolyse alcaline modérée par de l'hydroxyde de sodium aqueux 0,2 N, pour donner un glycoside précité de formule 1.

4. Procédé selon l'une des revendications 1 à 3, dans lequel on traite, à l'étape (v), le glycoside précité de formule 1, dissous dans un mélange de méthanol et de dioxane anhydres, par une solution de brome dans le chloroforme, pour fournir le dérivé bromo en position 14 correspondant, lequel est hydrolysé pendant deux jours à la température ambiante par une solution aqueuse de formiate de sodium, pour donner un glycoside précité de formule 2 sous la forme d'une base libre et, à l'étape (vi), on isole ledit glycoside de formule 2 sous la forme de son chlorhydrate.

5. Procédé de préparation d'un anthracycline glycoside de la formule 2 telle que définie à la revendication 1, à la condition que ni $R_2$ ni $R_3$ ne représentent un groupe amino, ou d'un de ses sels pharmaceutiquement acceptables d'addition avec un acide, ledit procédé comprenant :

(i') la condensation d'une aglycone protégée en position 14 de formule 6 :

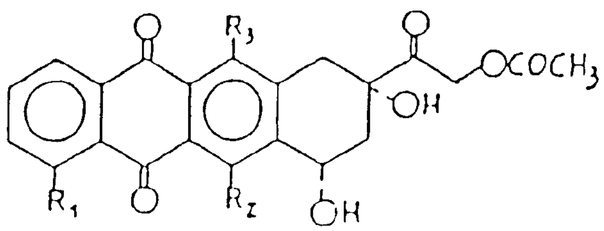

6

dans laquelle $R_1$, $R_2$ et $R_3$ sont tels que définis à la revendication 1, excepté que ni $R_2$ ni $R_3$ ne représentent un groupe amino, avec un halo-1 tétradésoxy-2,3,4,6 (N-trifluoro-acétamido)-4 L-érythro-hexopyranoside de formule 4 telle que définie à la revendication 1 ;

(ii') l'élimination du groupe protecteur en position 14 du glycoside N-protégé résultant de formule 7 :

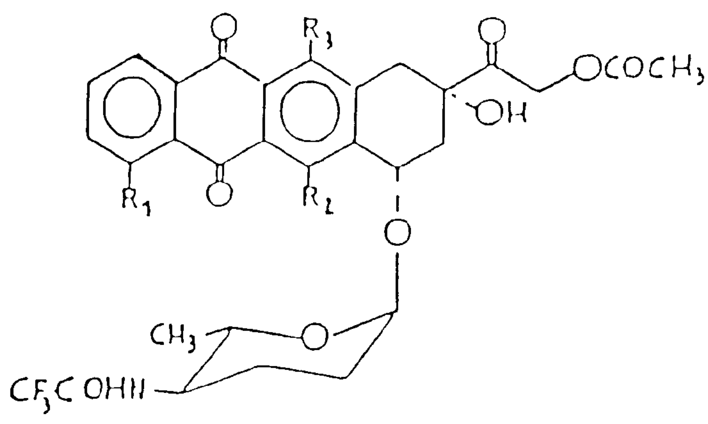

7

dans laquelle $R_1$, $R_2$ et $R_3$ sont tels que définis ci-dessus, pour donner le composé de formule 8 :

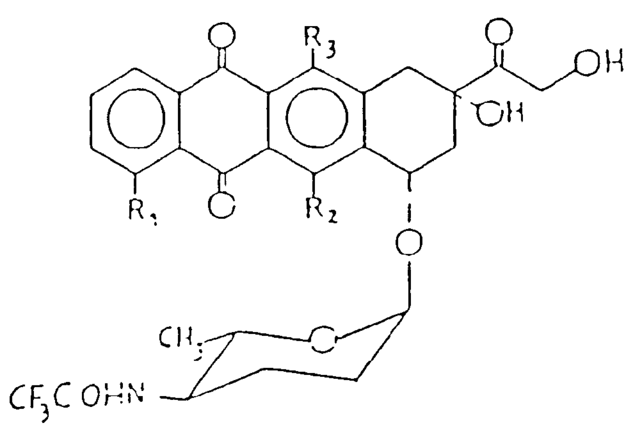

8

dans laquelle $R_1$, $R_2$ et $R_3$ sont tels que définis ci-dessus ;

(iii') la conversion du composé de formule 8 en un dérivé d'orthoformiate-9,14 de formule 9 :

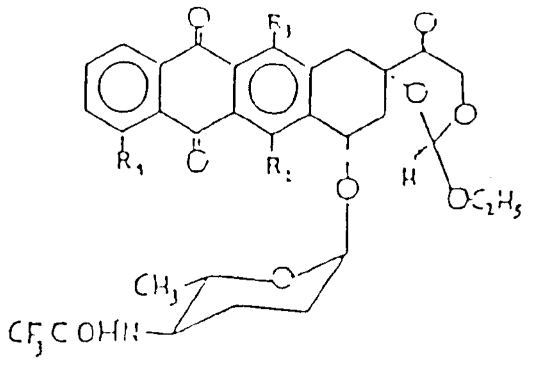

9

dans laquelle $R_1$, $R_2$ et $R_3$ sont tels que définis ci-dessus ;

(iv′) l'élimination du groupe N-trifluoroacétyle et du groupe protecteur orthoformiate, pour obtenir un glycoside précité de formule 2 ; et

(v′) si on le désire, la conversion dudit glycoside de formule 2 en l'un de ses sels pharmaceutiquement acceptables d'addition avec un acide.

6. Procédé selon la revendication 5, dans lequel, à l'étape (i′), on fait réagir, à la température ambiante, l'aglycone de formule 6 avec un chloro-1 hexapyranoside de formule 4, en présence d'un tamis moléculaire et de trifluorométhanesulfonate d'argent.

7. Procédé selon la revendication 5 ou 6, dans lequel, à l'étape (ii′), on traite le composé de formule 7, à 0°C, pendant quatre heures et sous atmosphère d'azote, par du carbonate de potassium.

8. Procédé selon l'une des revendications 5 à 7, dans lequel, à l'étape (iii′), on traite le composé de formule 8 par de l'orthoformiate de triéthyle.

9. Procédé selon la revendication 8, dans lequel on traite le composé de formule 8 par de l'orthoformiate de triéthyle dans du chlorure de méthylène et en présence de p-toluène sulfonate de pyridinium pendant une heure à la température ambiante.

10. Procédé selon l'une des revendications 5 à 9, dans lequel, à l'étape (iv′), on soumet le composé de formule 9 à une hydrolyse aqueuse alcaline modérée, pour éliminer le groupe N-trifluoroacétyle, et le traite par de l'acide acétique pour éliminer le groupe protecteur orthoformiate, afin d'obtenir ledit glycoside de formule 2 sous la forme d'une base libre, laquelle, à l'étape (v′), est traitée par du chlorure d'hydrogène méthanolique anhydre, pour isoler le glycoside sous la forme de son sel chlorhydrate.

11. Procédé de préparation d'un anthracycline glycosine de formule 2 selon la revendication 1, dans laquelle $R_1$ est tel que défini dans la revendication 1, et l'un parmi $R_2$ et $R_3$ représente hydroxy et l'autre parmi $R_2$ et $R_3$ représente un groupe amino, ou d'un de ses sels pharmaceutiquement acceptables d'addition avec un acide, ledit procédé comprenant :

(i″) la réduction du groupe nitro en C-6 ou C-11 d'un dérivé d'orthoformiate-9,14 de formule 9 telle que définie à la revendication 5, dans laquelle l'un parmi $R_2$ et $R_3$ représente hydroxy et l'autre parmi $R_2$ et $R_3$ représente un groupe nitro ;

(ii″) l'élimination du groupe N-trifluoroacétyle et du groupe protecteur orthoformiate à partir du composé contenant un groupe amino en C-6 ou C-11 ainsi formé, pour obtenir un glycoside précité de formule 2 ; et

(iii″) si on le désire, la conversion dudit glycoside de formule 2 en l'un de ses sels pharmaceutiquement acceptables d'addition avec un acide.

12. Procédé selon la revendication 11, dans lequel on effectue l'étape (i″) en portant au reflux le dérivé d'orthoformiate-9,14 de formule 9 dans un mélange de méthanol et de cyclohexène, en présence de Pd/C à 10% pendant dix minutes.

13. Procédé selon la revendication 11 ou 12, dans lequel on élimine le groupe N-trifluoroacétyle à l'étape (ii″) dans un milieu basique, et on élimine le groupe protecteur orthoformiate par de l'acide acétique, pour donner un diglycoside précité de formule 2, lequel est converti à l'étape (iii″) en son chlorhydrate, par traitement par du chlorure d'hydrogène méthanolique anhydre.